# EUROPEAN PATENT SPECIFICATION

(11) **EP 2 705 057 B1**
(45) Date of publication and mention of the grant of the patent: **03.08.2016**
(21) Application number: 12723733.7
(22) Date of filing: 08.05.2012
(51) Int. Cl.: C07K 16/22, C07K 16/46

(54) **THERAPEUTIC CANINE IMMUNOGLOBULINS AND METHODS OF USING THE SAME**
THERAPEUTISCHE IMMUNGLOBULINE FÜR HUNDE UND VERWENDUNGSVERFAHREN DAFÜR
IMMUNOGLOBULINES POUR THÉRAPIE CANINE ET LEURS PROCÉDÉS D'UTILISATION

(30) Priority: 06.05.2011 US 201161483481 P; 29.08.2011 GB 201114858; 06.09.2011 US 201161531439 P
(43) Date of publication of application: 12.03.2014
(73) Proprietor: Nexvet Australia Pty Ltd, Melbourne VIC 3000 (AU)
(72) Inventor: GEARING, David, Southbank Victoria 3006 (AU)
(74) Representative: Carr, Anne Marie
(86) International application number: PCT/GB2012/051008
(87) International publication number: WO 2012/153126

(56) References cited:
- WO-A2-01/77332
- WO-A2-2010/110838
- WO-A2-2010/117448
- PRESTA ET AL: "Engineering of therapeutic antibodies to minimize immunogenicity and optimize function", ADVANCED DRUG DELIVERY REVIEWS, ELSEVIER BV, AMSTERDAM, NL, vol. 58, no. 5-6, 7 August 2006 (2006-08-07), pages 640-656, XP024892146, ISSN: 0169-409X, DOI: 10.1016/J.ADDR.2006.01.026 [retrieved on 2006-08-07]

## Description

### Field of the Invention

The present invention relates to canine or canine derived antibodies, which have specific heavy chain constant regions, for use as antagonists of soluble extracellular mediators and/or cell surface receptors. The invention relates to the therapeutic use of the antibodies, or fragments thereof, in methods for the selective treatment of conditions such as inflammation, pain, cancer or infection in a canine subject.

### Background to the invention

Recombinant immunoglobulins and fusion proteins constructed using constant domain fragments of immunoglobulins are used to treat many human diseases including inflammatory diseases (e.g. rheumatoid arthritis, psoriasis, inflammatory bowel disease), allergies (e.g. asthma), cancers (e.g. lymphoma, breast cancer, bowel cancer), infectious diseases (e.g. RSV infection), pain (e.g. osteoarthritic pain, cancer pain, lower back pain) and eye disease (e.g. age-related macular degeneration).

The molecular targets for therapy include cytokines and chemokines (e.g. interleukin-1 (IL-1), interleukin-5 (IL-5), granulocyte colony-stimulating factor (GCSF), granulocyte-macrophage colony stimulating factor), growth factors (e.g. nerve growth factor (NGF), vascular endothelial cell growth factor (VEGF), tumour necrosis factor (TNF)), cell surface receptors (e.g. HER-2, VEGFR, EGFR, CD20), cell surface-bound growth factors (e.g. unprocessed tumour necrosis factor), viruses (e.g. RSV) and components of the complement cascade (e.g. C5). Many other targets that have evidence for involvement in disease processes are known (e.g. as described in the IMGT /MAb-DB database Version 1.3.1 14 Dec 2011 (URL: www.imgt.org/mAb-DB/query).

Native immunoglobulins are produced as different major subtypes, including immunoglobulin G (IgG), immunoglobulin A (IgA), immunoglobulin M (IgM) or immunoglobulin E (IgE) and in response to infection these immunoglobulins play various roles in pathogen recognition by binding to target antigens, neutralisation, destruction and removal. Immunoglobulin G is produced as several different isotypes (also known as isoforms), such as (in humans) IgG1, IgG2, IgG3 and IgG4. These antibody isotypes vary in structure, in particular with regard to differences in the amino acid sequences of the constant region, particularly around the hinge region of the constant domain (Fc) between the C1 and C2 domains.

Different antibody isotypes also differ in terms of the downstream effector functions which the antibody mediates. For example, the constant region sequence of an antibody can mediate a strong influence on characteristics such as effector functions (ADCC, complement fixing and activation), pharmacokinetics, and physical properties of an antibody. Antibodies having different isotypes also differ in terms of their ability to bind to IgG Fc receptors on immune cells. In humans, IgG1 and IgG3 are active in recruiting complement to aid in target destruction by the cascade of complement enzymes in the blood (CDC: complement-dependent cytotoxicity), and similarly IgG1 and IgG3 bind Fc receptors on immune cells that target the bound antigen for destruction by antibody-mediated cellular cytotoxicity (ADCC). By contrast, IgG2 and IgG4 do not recruit complement or activate ADCC mediated attack and simply bind to the target antigen with high affinity to inhibit or neutralise its activity.

Recombinant immunoglobulins and fusion proteins made from the same are designed to take into account the activity of the Fc isotype when considering the target for disease intervention. For example, it is preferable when considering a therapeutic approach which aims to use antibodies for the targeted killing of human cancer cells to construct the recombinant immunoglobulin from IgG1 or IgG3 isotype Fc domains, as the use of these isotypes will drive immune mediated destructive mechanisms such as CDC and ADCC. By contrast, when targeting soluble mediators in the context of sensitive human tissues, the Fc domain is either omitted (e.g. in treatment of human age-related macular degeneration Fab fragments targeting VEGF are preferred), or is constructed using IgG2 or IgG4 Fc domains (e.g. targeting nerve growth factor in the context of neuropathic or inflammatory pain, or complement C5 in nephritis, psoriasis or rheumatoid arthritis). These considerations also apply to immunoglobulin fusion proteins, such as soluble TNF receptor Fc fusion proteins in the treatment of conditions such as rheumatoid arthritis, which are based on human IgG1 Fc therapeutics.

In canines and other species such as mice and horses, immunoglobulin isoforms also exist but have insufficient homology between one another to determine *a priori* which sequence will be active or inactive in inducing downstream effector functions such as CDC or ADCC. Furthermore, the number of immunoglobulins varies between species (e.g. in dog there are four IgG immunoglobulins, these being defined as caIgG-A, caIgG-B, caIgG-C, and caIgG-D (Tang et al., 2001). In horses, there are seven IgG isotypes (Wagner, 2006).

It is not possible to determine from sequence analysis or sequence homology alone whether a specific immunoglobulin isotype of a non-human species will be active or inactive in terms of mediating Fc receptor binding and downstream effector function. However, if these were known, it would be of significant value as the choice of isotype constant regions for antibody generation can be critical in order to provide the therapeutic effectiveness of an antibody or antibody based therapeutics, such as an antibody binding fragment or fusion protein.

WO 2010/117448 and WO0177332 disclose recombinant antibodies comprising one or more canine sequences, and methods for their preparation and use. In some embodiments, recombinant antibodies are provided that have improved Fc-receptor binding and/or improved ADCC effector function.

### Summary of the Invention

Following extensive experimentation, it has been surprisingly identified by the present inventor that the isotypes of canine IgG immunoglobulin share the characteristics observed in human IgG antibodies that certain IgG antibody isotypes are active in terms of activating immune effector functions, while other IgG antibody isotypes do not activate immune effector functions and are accordingly inactive. Furthermore, of the four known canine heavy chain immunoglobulins (known as HCA (caIgG-A), HCB (caIgG-B), HCC (caIgG-C) and HCD (caIgG-D)), the inventor has surprisingly identified that heavy chain constant domains from two (caIgG-B and caIgG-C) of the four canine heavy chain immunoglobulins, when constructed as various recombinant forms targeting different therapeutic targets, surprisingly bind complement, whereas the other two (caIgG-A and caIgG-D) do not.

Accordingly, the present invention defines certain recombinant canine immunoglobulins, or fusion proteins made therefrom, which are used in the therapy of canines where target destruction is desired (e.g. in cancer or infectious disease treatment); and certain other isoforms which may be preferred for therapeutic treatments in canines where target neutralisation alone, rather than target destruction, is desired (e.g. in the treatment of pain).

The present disclosure provides recombinant canine immunoglobulins that can be distinguished by their ability or otherwise to bind to the first component of the complement cascade, based on the isotype of their heavy chain constant domain. As a result, and for the first time, recombinant canine immunoglobulins can be selected according to their intended use in treatment of disease in canines, whether for purposes where the intended target is selected for immune mediated destruction through complement mediated cytotoxicity (CDC; e.g. for use in killing canine tumours in vivo) or where the target is selected simply for neutralisation in the absence of undesirable immune mediated destruction (e.g. in the proximity of nerves, or in the eye).

According to a first aspect of the invention there is provided an antibody, fusion protein or a binding fragment thereof for use in the therapeutic treatment of a canine where target neutralisation is desired in the absence of undesirable effector function, wherein said antibody, fusion protein or binding fragment has a heavy chain constant domain comprising the amino acid sequence of SEQ ID NO:8, SEQ ID NO:11 or SEQ ID NO:13, wherein the amino acid sequence of the heavy chain minimises the activation of downstream immune system effector functions when the antibody, fusion protein or binding fragment is bound to its target antigen.

In certain embodiments, the therapeutic treatment of the canine relates to the treatment of pain or inflammation or a condition associated therewith, such as arthritis or an arthritic condition.

Disclosed herein is use of an antibody, fusion protein or a binding fragment thereof comprising a heavy chain constant domain having the amino acid sequence of SEQ ID NO:8, SEQ ID NO:11 or SEQ ID NO:13, wherein the amino acid sequence of the heavy chain minimises the activation of downstream immune system effector functions when the antibody, fusion protein or binding fragment is bound to its target antigen, in the preparation of a medicament for use in the treatment of pain or inflammation in a canine subject or a condition associated therewith, such as arthritis or an arthritic condition.

Also disclosed is a method for treating, inhibiting or ameliorating pain or inflammation or a condition associated therewith, such as arthritis or an arthritic condition, in a canine subject in need thereof, the method comprising the steps of:
- providing an antibody, fusion protein or a binding fragment thereof which binds specifically to a target antigen which has a specific function in the treatment or prevention of pain or inflammation, wherein the antibody, fusion protein or binding fragment thereof has a heavy chain constant domain comprising the amino acid sequence of SEQ ID NO:8, SEQ ID NO:11 or SEQ ID NO:13 and wherein the antibody, fusion protein or binding fragment thereof does not activate downstream immune system effector functions, and
- administering a therapeutically effective amount of the antibody, fusion protein or binding fragment thereof to the canine subject.

Also disclosed is a canine derived antibody, fusion protein or a binding fragment thereof which has a heavy chain constant domain comprising the amino acid sequence of SEQ ID NO:8, SEQ ID NO:11 or SEQ ID NO:13 for use in the preparation of a medicament for the treating, inhibiting or ameliorating pain or inflammation in a canine subject.

In certain embodiments of the first aspect of the invention, the pain is neuropathic pain. In particular, the pain may be peri-operative, post-operative or post-surgical pain. Post-operative pain may result following any operating procedure which in canines may include, but is not limited to, orthopaedic surgery, soft tissue surgery, ovariohysterectomy procedures, castration procedures and the like. In certain further embodiments, the pain is chronic pain associated with cancer or a cancerous condition (oncologic pain). In certain further embodiments, the pain is associated with, or resulting from, rheumatoid arthritis, osteoarthritis, inflammation or pruritis.

Also disclosed is a method for the treatment of arthritis or an arthritic condition in a canine subject, the method comprising the steps of:
- providing an antibody, fusion protein or a binding fragment thereof which binds specifically to a target antigen which has a specific function in the treatment or prevention of pain or inflammation, wherein the antibody, fusion protein or binding fragment thereof has a heavy chain constant domain comprising the amino acid sequence of SEQ ID NO:8, SEQ ID NO:11 or SEQ ID NO:13 and wherein the antibody, fusion protein or binding fragment thereof does not activate downstream immune system effector functions, and
- administering a therapeutically effective amount of the antibody, fusion protein or binding fragment thereof to the canine subject in need of such treatment.

The methods described herein may further comprise the step of co-administering at least one further agent which may enhance and/or complement the effectiveness of the antibody. For example, the antibody or antigen binding fragment thereof may be co-administered along with at least one analgesic, NSAID, opioid, corticosteroid or steroid. Examples of suitable analgesics include, but are not limited to butorphanol, 10 buprenorphine, fentanyl, flunixin meglumine, merpidine, morphine, nalbuphine and derivatives thereof. Suitable NSAIDS include, but are not limited to acetaminophen, acetylsalicylic acid, carprofen, etodolac, ketoprofen, meloxicam, firocoxib, robenacoxib, deracoxib and the like.

The foregoing methods may be accompanied by the administration of at least one further agent. Said agent may be a therapeutically active agent which may be one or more of the group selected from: an antibiotic, antifungal, antiprotozoal, antiviral or similar therapeutic agents. Furthermore the at least one further agent may be an inhibitor of mediator(s) of inflammation such as a PGE-receptor antagonist, an immunosuppressive agent, such as cyclosporine, an anti-inflammatory glucocorticoids. The at least one further agent may be an agent which is used for the treatment of cognitive dysfunction or impairment, such as memory loss or related conditions which may become increasingly prevalent in older canines. Further still, the at least one further agent may be an anti-hypertensive or other compound used for the treatment of cardiovascular dysfunction, for example to treat hypertension, myocardial ischemia, congestive heart failure and the like. Further still, the at least one further agent may be a diuretic, vasodilator, beta-adrenergic receptor antagonist, angiotensin-II converting enzyme inhibitor, calcium channel blocker and HMG-CoA reductase inhibitor.

In certain embodiments of the foregoing aspect of the invention, the downstream immune system effector functions are selected from the group comprising complement dependent cytotoxicity (CDC), antibody dependent cell mediated cytotoxicity (ADCC), and antibody dependent cellular pathogenesis (ADCP). In specific embodiments, the amino acid sequence of the heavy chain constant domain inhibits binding of the heavy chain to C1q, this preventing induction of the complement cascade and complement dependent cytotoxicity (CDC).

In certain embodiments, the target antigen is a soluble mediator. In certain embodiments, the target antigen is nerve growth factor (NGF). In certain embodiments, the antibody specifically binds to and antagonises a receptor which mediates pain or inflammation. In certain further embodiments, the target antigen can be selected from the group consisting of, but not limited to, cytokines or chemokines (e.g. interleukin-1 and related interleukins IL-2 through IL-35, granulocyte colony-stimulating factor, granulocyte-macrophage colony stimulating factor, erythropoietin, thrombopoetin, leukaemia inhibitory factor, ciliary neurotrophic factor, oncostatin M), growth factors (e.g. nerve growth factor (NGF), brain-derived neurotrophic factor (BDNF), neurotrophin-3, neurotrophin-4, vascular endothelial cell growth factor (VEGF), tumour necrosis factor (TNF), cell surface receptors (e.g. HER-2, VEGFR, EGFR, CD20), cell surface-bound growth factors (e.g. unprocessed tumour necrosis factor), viruses (e.g. RSV) and components of the complement cascade (e.g. C5, C5a).

According to yet further aspect of the invention there is provided an antibody, fusion protein or a binding fragment thereof for use in the therapeutic treatment of a canine where target destruction is desired, wherein said antibody, fusion protein or binding fragment has a heavy chain constant domain comprising the amino acid sequence of SEQ ID NO:9, SEQ ID NO:10, or SEQ ID NO:14 wherein the amino acid sequence of the heavy chain mediates the activation of downstream immune system effector functions when the antibody, fusion protein or binding fragment is bound to its target antigen.

In certain embodiments, the therapeutic treatment of the canine relates to the treatment of a cancerous or malignant condition.

Disclosed herein is use of an antibody, fusion protein or a binding fragment thereof comprising a heavy chain constant domain having the amino acid sequence of SEQ ID NO:9, SEQ ID NO:10 or SEQ ID NO:14, wherein the amino acid sequence of the heavy chain mediates the activation of downstream immune system effector functions when the antibody, fusion protein or binding fragment is bound to its target antigen, in the preparation of a medicament for use in the treatment of a cancerous or malignant condition in a canine subject.

Also disclosed is a method for the treatment or prevention of a cancerous or malignant condition in a canine subject, the method comprising the steps of:
- providing an antibody, fusion protein or a binding fragment thereof which binds specifically to a target antigen which has a specific function in the treatment of a cancerous or malignant condition, wherein the antibody, fusion protein or binding fragment has a heavy chain constant domain comprising the amino acid sequence of SEQ ID NO:9, SEQ ID NO:10 or SEQ ID NO:14 and wherein the antibody, fusion protein or binding fragment activates downstream immune system effector functions, and
- administering a therapeutically effective amount of the antibody, fusion protein or binding fragment to the canine subject in need of such treatment.

The foregoing method may further comprise the step of co-administering at least one further agent which may enhance and/or complement the effectiveness of the antibody disclosed herein.

Also disclosed is use of a canine derived antibody, fusion protein or a binding fragment thereof, which has a heavy chain constant domain comprising the amino acid sequence of SEQ ID NO:9, SEQ ID NO:10 or SEQ ID NO:14 in the preparation of a medicament for the treatment of cancerous or malignant condition in a canine subject.

In certain embodiments of the foregoing aspect of the invention, the downstream immune system effector functions are selected from the group comprising complement dependent cytotoxicity (CDC), antibody dependent cell mediated cytotoxicity (ADCC), and antibody dependent cellular pathogenesis (ADCP). In specific embodiments, the amino acid sequence of the heavy chain constant domain provides for binding of the heavy chain to C1q, this preventing induction of the complement cascade and complement dependent cytotoxicity (CDC). In certain embodiments, the heavy chain constant domain provides for binding to Fc receptors, which may in turn mediate ADCP and/or ADCC immune responses.

In certain embodiments, the target antigen is a cancer specific antigen. In certain further embodiments of the invention, the target antigen may be selected from the group of membrane bound proteins expressed on canine tumour cells. In further embodiments of the invention, the membrane bound canine tumour proteins may be selected from the group of proteins including CD2, CD4, CD8, CD20, EGFR, VEGFR, HER2 and the like. In certain further embodiments, the target antigen can be selected from the group consisting of, but not limited to, cytokines and chemokines (e.g. interleukin-1 (IL-1), IL-2, IL-3 and interleukins numerically through to IL-35, granulocyte colony-stimulating factor, granulocyte-macrophage colony stimulating factor, erythropoietin, thrombopoetin, leukaemia inhibitory factor, ciliary neurotrophic factor, oncostatin M), growth factors (e.g. nerve growth factor (NGF), brain-derived neurotrophic factor (BDNF), neurotrophin-3, neurotrophin-4, vascular endothelial cell growth factor (VEGF), tumour necrosis factor (TNF)), cell surface receptors (e.g. HER-2, VEGFR, EGFR, CD20), cell surface-bound growth factors (e.g. unprocessed tumour necrosis factor), viruses (e.g. RSV) and components of the complement cascade (e.g. C5, C5a).

Also disclosed is an antibody, fusion protein or a binding fragment thereof for use in the treatment of a condition in a canine, wherein the antibody, fusion protein or binding fragment has a heavy chain constant domain which does not bind to C1q when the antibody, fusion protein or binding fragment is bound to its target antigen and wherein the antibody, fusion protein or binding fragment can be purified using Protein A chromatography.

The antibody, fusion protein or binding fragment may have a heavy chain constant domain comprising the amino acid sequence of SEQ ID NO:13. Alternatively, said antibody, fusion protein or binding fragment may have a heavy chain constant domain selected from SEQ ID NO:12 and SEQ ID NO:15 or may have a heavy chain of SEQ ID NO:6.

Also disclosed is use of an antibody, fusion protein or a binding fragment which comprises a heavy chain constant domain which does not bind to C1q when the antibody, fusion protein or binding fragment is bound to its target antigen and wherein the antibody, fusion protein or binding fragment can be purified using Protein A chromatography in the preparation of a medicament for the treatment of a condition associated with pain and/or inflammation in a canine.

Said antibody, fusion protein or binding fragment may have a heavy chain constant domain comprising the amino acid sequence of SEQ ID NO:13. Alternatively, said antibody, fusion protein or binding fragment may have a heavy chain constant domain selected from SEQ ID NO:12 and SEQ ID NO:15 or may have a heavy chain of SEQ ID NO:6.

Also disclosed is a method for treating, inhibiting or ameliorating pain or inflammation or a condition associated therewith, such as arthritis or an arthritic condition, in a canine subject in need thereof, the method comprising the steps of:
- providing an antibody, fusion protein or binding fragment which comprises a heavy chain constant domain which does not bind to C1q when the antibody is bound to its target antigen and wherein the antibody can be purified using Protein A chromatography, and
- administering a therapeutically effective amount of the antibody, fusion protein or binding fragment thereof to the canine subject.

Said antibody, fusion protein or binding fragment may have a heavy chain constant domain comprising the amino acid sequence of SEQ ID NO:13. Alternatively, said antibody, fusion protein or binding fragment may have a heavy chain constant domain selected from SEQ ID NO:12 and SEQ ID NO:15 or may have a heavy chain of SEQ ID NO:6.

Also disclosed are: (i) nucleic acids which encode any of the foregoing antibodies, fusion proteins or antibody fragments, (ii) vectors which carry said nucleic acids, (iii) host cells carrying said vectors. Also disclosed are methods for producing antibodies and fusion proteins as disclosed herein. Further disclosed are pharmaceutical compositions which comprise the antibodies or fusion proteins disclosed herein along with at least one carrier, diluent or excipient.

Disclosed herein is a recombinant antibody, fusion protein or binding fragment thereof which can be therapeutically administered to a canine in order to specifically bind to a target antigen and which further mediates an immune response which is characterised by C1q complement binding to said antibody, fusion protein or binding fragment thereof and associated complement dependent cytotoxicity, wherein the heavy chain of the antibody, fusion protein or binding fragment thereof comprises canine immunoglobulin heavy chain constant domain isotype B (HCB, caIgG-B) having an amino acid sequence of SEQ ID NO:9, canine immunoglobulin heavy chain constant domain isotype C (HCC, caIgG-C) having an amino acid sequence of SEQ ID NO:10 or aglycosyl canine immunoglobulin heavy chain isotype C (HCC, caIgG-C) having an amino acid sequence of SEQ ID NO:14.

Also disclosed is use of an antibody, fusion protein or binding fragment thereof which comprises canine immunoglobulin heavy chain constant domain isotype B (HCB, caIgG-B) having an amino acid sequence of SEQ ID NO:9, canine immunoglobulin heavy chain constant domain isotype C (HCC, caIgG-C) having an amino acid sequence of SEQ ID NO:10 or aglycosyl canine immunoglobulin heavy chain isotype C (HCC, caIgG-C) having an amino acid sequence of SEQ ID NO:14 in the preparation of a medicament for use in the treatment of a condition where specific binding to a target antigen is required and where an immune response which is characterised by C1q complement binding to said antibody, fusion protein or binding fragment thereof and associated complement dependent cytotoxicity is desirable.

Also disclosed is a method for the treatment of a cancerous condition in a canine subject, the method comprising the step of:
- providing an immunoglobulin, fusion protein or a binding fragment thereof which has binding specificity for a tumour specific antigen and which further comprises canine immunoglobulin heavy chain constant domain isotype B (HCB, caIgG-B) having an amino acid sequence of SEQ ID NO:9, canine immunoglobulin heavy chain constant domain isotype C (HCC, caIgG-C) having an amino acid sequence of SEQ ID NO:10 or aglycosyl canine immunoglobulin heavy chain isotype C (HCC, caIgG-C) having an amino acid sequence of SEQ ID NO:14, and
- administering a therapeutically effective amount of the immunoglobulin, fusion protein or binding fragment to the canine subject in need thereof.

Also described herein are antibodies or fusion proteins which bind to a desired target antigen and which comprise heavy chain constant domains which do not bind C1q and which accordingly do not mediate an immune response involving complement dependent cytotoxicity.

Described herein is a recombinant antibody, fusion protein or binding fragment thereof which can be therapeutically administered to a canine in order to specifically bind to a target antigen, wherein the constant domain of the antibody or fusion protein does not bind to C1q complement and wherein the heavy chain of the constant domain comprises canine immunoglobulin heavy chain constant domain isotype A (HCA, caIgG-A) having an amino acid sequence of SEQ ID NO:8, canine immunoglobulin heavy chain constant domain isotype D (HCD, caIgG-D) having an amino acid sequence of SEQ ID NO:11 or an aglycosyl canine immunoglobulin heavy chain constant domain isotype B having an amino acid sequence of SEQ ID NO:13 (HCB*, caIgG-B).

Also described is use of an antibody, fusion protein or binding fragment thereof which comprises canine immunoglobulin heavy chain constant domain isotype A (HCA, caIgG-A) having an amino acid sequence of SEQ ID NO:8, canine immunoglobulin heavy chain constant domain isotype D (HCD, caIgG-D) having an amino acid sequence of SEQ ID NO:11 or an aglycosyl canine immunoglobulin heavy chain constant domain isotype B having an amino acid sequence of SEQ ID NO:13 (HCB*, caIgG-B) in the preparation of a medicament for use in the treatment of a condition where specific binding to a target antigen is required and where an immune response which is characterised by C1q complement binding to said antibody, fusion protein or binding fragment thereof and associated complement dependent cytotoxicity is not desirable.

Further described is a method for the treatment of a condition in a canine subject, the method comprising the step of:
- providing an immunoglobulin, fusion protein or a binding fragment thereof which has binding specificity for a tumour specific antigen and which further comprises canine immunoglobulin heavy chain constant domain isotype A (HCA, caIgG-A) having an amino acid sequence of SEQ ID NO:8, canine immunoglobulin heavy chain constant domain isotype D (HCD, caIgG-D) having an amino acid sequence of SEQ ID NO:11 or an aglycosyl canine immunoglobulin heavy chain constant domain isotype B having an amino acid sequence of SEQ ID NO:13 (HCB*, caIgG-B), and
- administering a therapeutically effective amount of the immunoglobulin, fusion protein or binding fragment to the canine subject in need thereof.

The aglycosylated constant domain may be designed for antibody construction in the absence of CDC activity is alanine-substituted aglycosylated heavy chain HCB having an amino acid sequence of SEQ ID NO:6 (denoted HCB*). The antibodies incorporating heavy chains HCA, HCD or CDC inactive aglycosylated forms of HCA, HCB or HCD (HCA*, HCB* or HCD*: SEQ ID NO:12, SEQ ID NO:13 and SEQ ID NO:15) may be directed to canine growth factors, hormones, cytokines, chemokines or other soluble mediators such as components of the complement cascade. The antibodies incorporating heavy chains HCA, HCD or HCB* may be directed to canine nerve growth factor (NGF) for the purposes of neutralising canine NGF biological activity in a canine, without inducing CDC.

In certain embodiments of the foregoing aspects of the invention the antibody is a monoclonal antibody. In certain further embodiments, the antibody is a chimeric antibody. In some embodiments, the antibody is a caninised antibody, that is, an antibody which has an amino acid sequence which has been de-immunised such that neutralising antibodies will not be produced there against when administered to a canine subject. Typically the heavy chain constant domains of the antibody are selected or modified by way of amino acid substitution or deletion such that the constant domains do not mediate downstream effector functions. In certain embodiments, the antibody may be conjugated to at least one reporter molecule.

In certain further embodiments at least one residue in the constant domain of the antibodies or fusion proteins for use in the foregoing aspects of the invention can be substituted or deleted in order to prevent the glycosylation of that residue. In a further aspect of the invention the canine immunoglobulin heavy chain constant domain may be fused whole or in part to the extracellular domain of a cytokine or chemokine receptor or other trans-membrane protein (e.g. the TNF receptor), whereby the whole or fragment of the canine immunoglobulin heavy chain constant domain is selected from the group HCA, HCD or HCB* where it is desired that the canine extracellular domain-immunoglobulin heavy chain fusion protein does not activate CDC (e.g. where TNF receptor Fc fusion proteins are designed for the neutralization of soluble TNF) or conversely, the canine immunoglobulin heavy chain constant domain is selected from the group HCB, HCC or HCC* where it is desired that the canine extracellular domain-immunoglobulin heavy chain fusion protein (e.g. where TNF receptor Fc fusion proteins are designed to kill membrane-associated TNF bearing inflammatory cells).

In further aspects of the invention, the canine receptor-Fc fusion proteins may be selected from the group of extracellular domains of membrane bound receptors found on canine cells fused to canine immunoglobulin domain heavy chain Fc regions. In a further aspect of the invention, the antibodies incorporating heavy chains HCB, HCC or HCC* are directed to CD20 for the purposes of inducing CDC of canine CD20 expressing cells, such as canine lymphoma cells through the binding of these antibodies to CD20 on their surface.

Furthermore, it is preferred that the caninised antibodies are not cross-reactive to any other epitopes present in canines, and further that neutralising antibodies are not generated against the antibodies disclosed herein when they are administered to a canine.

In certain further embodiments, modifications to the amino acid sequence of the constant regions of the heavy chain may be made to the antibodies or fusion proteins described herein. Said modification may involve the addition, substitution or deletion of one or more amino acid residues. Said amino acid changes are typically performed in order to modify the functional characteristics of the antibody. For example, amino acid modification may be performed to prevent downstream effector functions mediated by the antibody constant domains, for example by preventing the ability of the antibody to bind to Fc receptors, activate complement or induce ADCC. Furthermore, modifications may be made to the amino acid residues of the hinge region of the heavy chain constant domain in order to modify the circulatory half-life of an antibody when it is administered to a canine.

Described herein are multi-specific or multivalent antibodies comprising an antibody or binding fragment as disclosed herein coupled or conjoined to other antibodies with different binding specificities for use in combination therapy. A multi-specific antibody comprises at least one antibody or binding fragment specific to a first epitope, and at least one binding site specific to another epitope present on the antigen, or to a different antigen. A multivalent antibody comprises antibodies or antibody binding fragments which have binding specificity to the same epitope. Accordingly, described herein is an antibody fusion protein comprising four or more Fv regions or Fab regions of the antibodies disclosed herein. Also described is a bispecific antibody, wherein an antibody or binding fragment thereof as disclosed herein is linked to a second antibody or binding fragment thereof which has binding specific for a second target, said target not being the first antigen. Such multivalent, bispecific or multispecific antibodies can be made by a variety of recombinant methods which would be well known to the person skilled in the art.

In certain embodiments, the antibody, fusion protein or antigen binding fragment is administered to the canine at a dose ranging from about 0.01 mg/kg of body weight to about 10 mg/kg of body weight, in particular from 0.03 mg/kg of body weight to about 3 mg/kg of body weight.

Also disclosed is a composition comprising an antibody, fusion protein or binding fragment thereof as disclosed herein. The composition may further comprise at least one pharmaceutically acceptable carrier. The composition may further comprise at least one analgesic, NSAID, opioid, corticosteroid or steroid.

Also disclosed is an isolated nucleic acid which encodes the antibody, fusion protein or antibody binding fragments described herein. Accordingly, described herein is an isolated nucleic acid that encodes an antibody, fusion protein or antigen-binding fragment as disclosed herein. The isolated nucleic acid may further encode one or more regulatory sequences operably linked thereto.

Also disclosed is an expression vector comprising a polynucleotide encoding a heavy and/or light chain variable domain or a heavy and/or light chain constant domain as described herein. The expression vector may further comprise one or more regulatory sequences. The vector may be a plasmid or a retroviral vector. Also described is a host cell incorporating the expression vector disclosed herein. Further described is a host cell which produces the antibody described herein.

Also described is a method for producing an antibody or fusion protein as described herein, the method comprising the step of culturing the host cell described herein to allow the cell to express the antibody. Also described is a method of producing the antibody or fusion protein described herein comprising the steps of expressing one or more of the polynucleotides / nucleic acids or vectors described herein which express the light and/or heavy chains of the antibodies disclosed herein in a suitable host cell, recovering the expressed polypeptides, which may be expressed together in a host cell, or separately in different host cells, and isolating antibodies. Also described is a method for treating, ameliorating or inhibiting pain in a canine, the method comprising the step of administering to the canine an effective amount of a polynucleotide which encodes an antibody or fusion protein having a heavy chain constant domain comprising the amino acid sequence of SEQ ID NO:8-SEQ ID NO:11.

### Canine antibody purification

In the case of those canine antibodies where target neutralisation is desired in the absence of unwanted immune effector activity, the present inventor has surprisingly discovered that native isoforms of canine heavy chains that lack CDC activity also bind Staphylococcus Protein A very poorly, if at all, and consequently this common method for purification of antibodies cannot be used in manufacture. The inventor describes three ways of overcoming this restriction, through the use of alternative purification strategies and through mutation of the heavy chain to prevent glycosylation during production. Purified antibodies prepared by these methods were produced by the inventor and shown to have the desirable properties of being safe and effective *in-vivo* in dogs without unwanted immunogenicity.

Disclosed herein is a method for the purification of a canine derived immunoglobulin or an immunoglobulin or fusion protein comprising a canine heavy chain constant domain of isotype A (HCA, caIgG-A) having an amino acid sequence of SEQ ID NO:8 or a canine immunoglobulin heavy chain constant domain of isotype D (HCD, caIgG-D) having an amino acid sequence of SEQ ID NO:11 from a source mixture, the method comprising the steps of:
(i) providing a source mixture comprising target immunoglobulins or fusion proteins,
(ii) subjecting the source mixture to anion exchange chromatography;
(iii) subjecting the source mixture to hydrophobic interaction chromatography; and
(iv) subjecting the source mixture to size exclusion chromatography.

The method may comprise the step of buffer exchange in phosphate buffered saline. Typically the method produces a purified antibody which is fractionated to high purity and bioactivity.

Disclosed herein is a method of production of an aglycosylated canine antibody that can be purified by Protein A chromatography.

Also described is a canine or canine derived antibody or fusion protein produced in accordance with any of the methods defined herein, for use in the therapeutic treatment of a canine. Also described is the use of an anti-canine NGF antibody in the preparation of a medicament for use in treating an immune mediated condition, or a condition associated with pain, in a canine.

Also described is a method for the purification of a canine derived immunoglobulin or an immunoglobulin or fusion protein comprising a canine heavy chain constant domain of isotype A (HCA, caIgG-A) having an amino acid sequence of SEQ ID NO:8 or a canine immunoglobulin heavy chain constant domain of isotype D (HCD, caIgG-D) having an amino acid sequence of SEQ ID NO:11 from a source mixture, the method comprising the steps of:
(i) providing a source mixture comprising target immunoglobulins,
(ii) subjecting the source mixture to captoadhere affinity chromatography; and
(iii) subjecting the source mixture to anion exchange chromatography.

Also described is an antibody or fusion protein produced from the purification method described herein for use in the treatment of a canine.

### Brief Description of the Figures

Figures 1A and 1B show equivalent binding of anti-NGF antibodies (expressed into the supernatant of transfected CHO cells) constructed using four different isotypes of canine heavy chains (HCA, HCB, HCC, HCD) to murine NGF by ELISA (Figure 1A) and equivalent inhibition of NGF proliferation of TF-1 cells (Figure 1B).
Figure 2 shows differential binding of complement to NGF-bound anti-canine NGF antibody isotypes as measured by anti-C1q ELISA.
Figures 3a and 3b show binding of NGF-captured glycosylated and aglycosylated caninised anti-NGF monoclonal antibodies to complement as measured by anti-C1q ELISA.

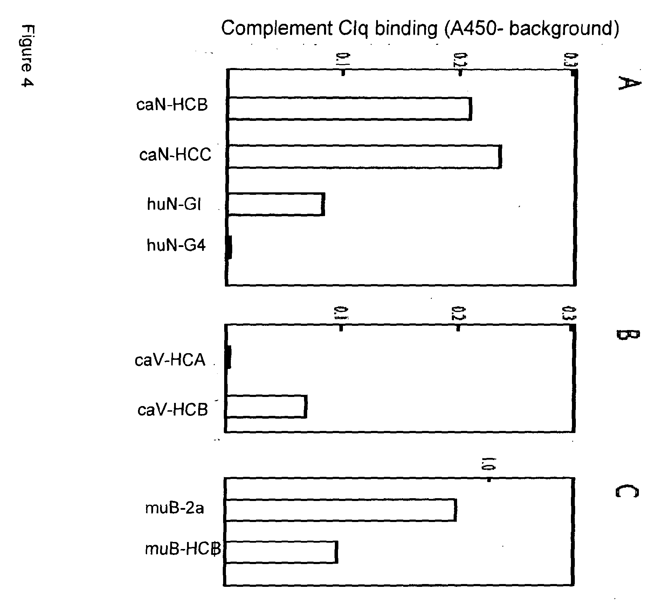

Figure 4 shows the binding of NGF-captured caninised anti-NGF monoclonal antibodies (MAbs), anti-canine VEGF MAbs and anti-human CD20/ canine HCB chimeric MAb to complement measured by anti-C1q ELISA. In particular, Figures 4A, 4B and 4C show binding of complement to antibodies constructed using various canine heavy chain isotypes. Caninised monoclonal antibodies (MAb) were expressed in CHO cells and tested for their ability to bind complement C1q. Panel A- caninised anti-NGF antibodies (caN-HCB, caN-HCC) compared with humanised antibody isotypes (huN-G1, huN-G4); Panel B- anti-VEGF antibodies constructed with canine HCA (caV-HCA) and HCB (caV-HCB) isotypes; Panel C- anti-CD20 antibody constructed using HCB isotype (mub-HCA) compared with mouse IgG2a isotype (muB-2a).

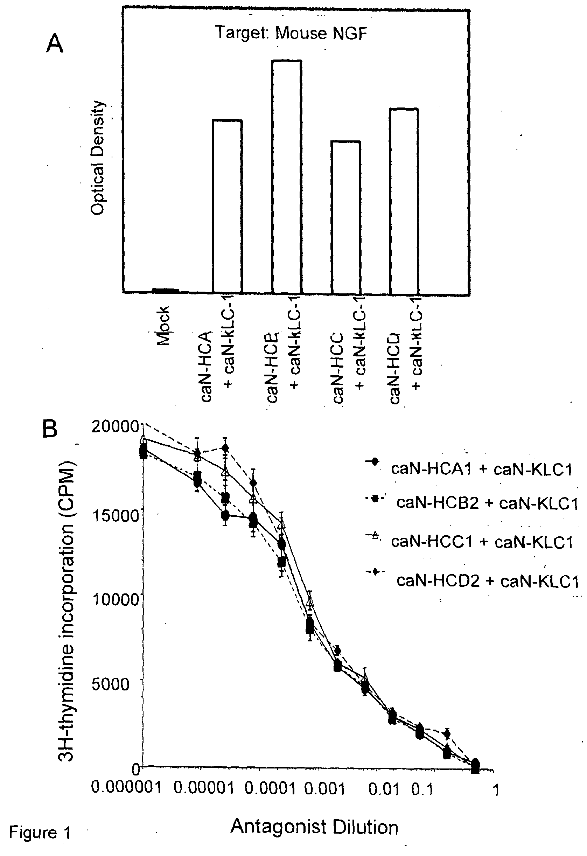

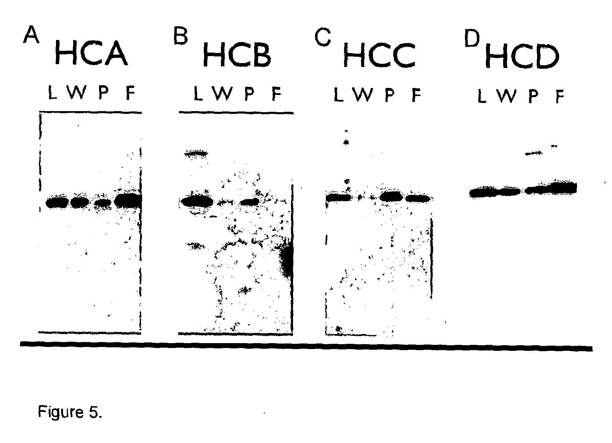

Figure 5 shows relative recovery of anti-NGF antibody isotypes purified by Protein A and detected using anti-canine polyclonal immunoglobulin by Western blot. The supernatants from Figure 1 were passed over Protein A columns and specifically bound material eluted. Equal volumes of eluate were subjected to SDS-PAGE. Canine isotypes HCA, HCC and HCD bound weakly to Protein A as indicated by significant material in the wash and flow through fractions. Figures A-D show relative recovery of canine antibody isotypes HCA, HCB, HCC and HCD by Protein A: L, load; W, wash; P, purified; F, flow through. Anti-NGF antibody supernatants from Figure 1 were used in this experiment.

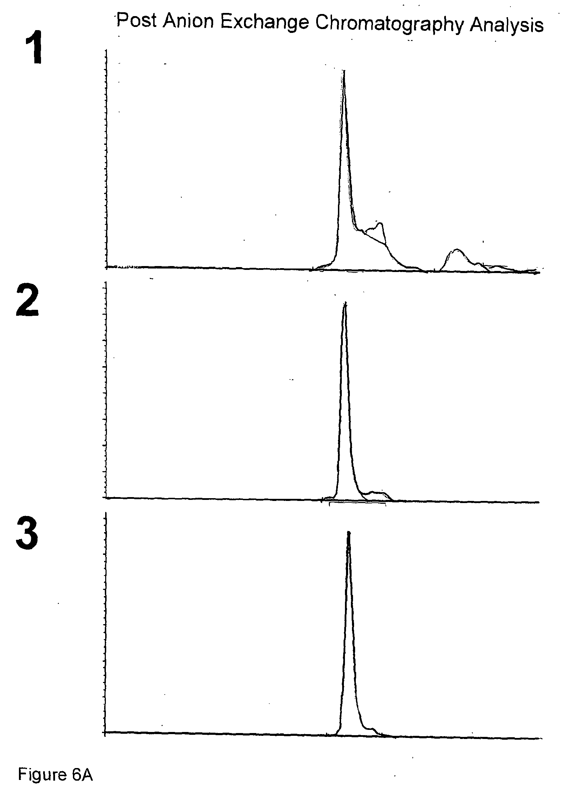

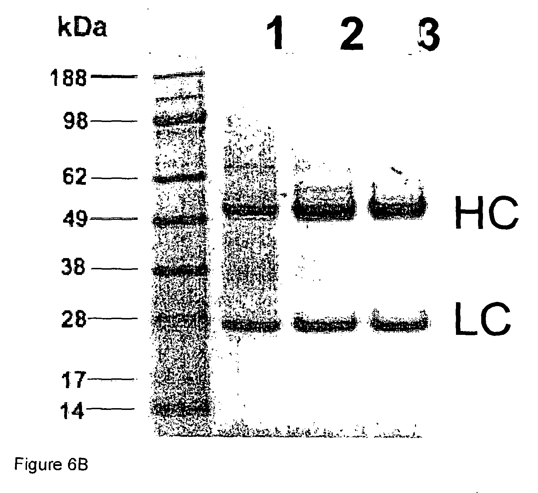

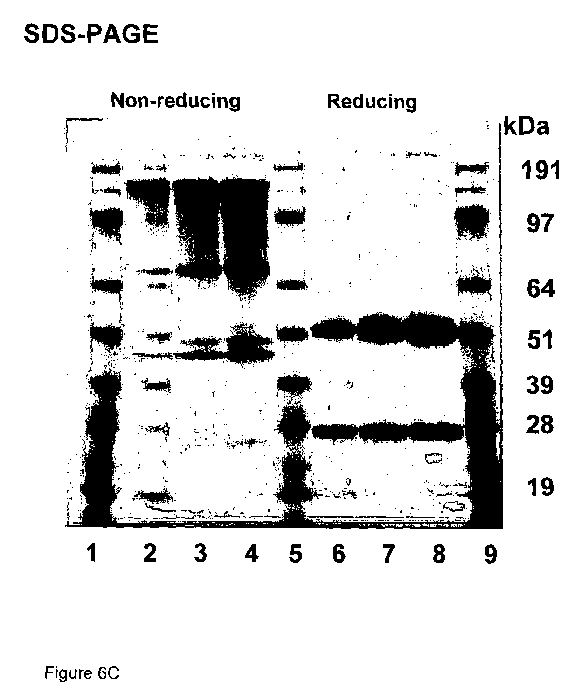

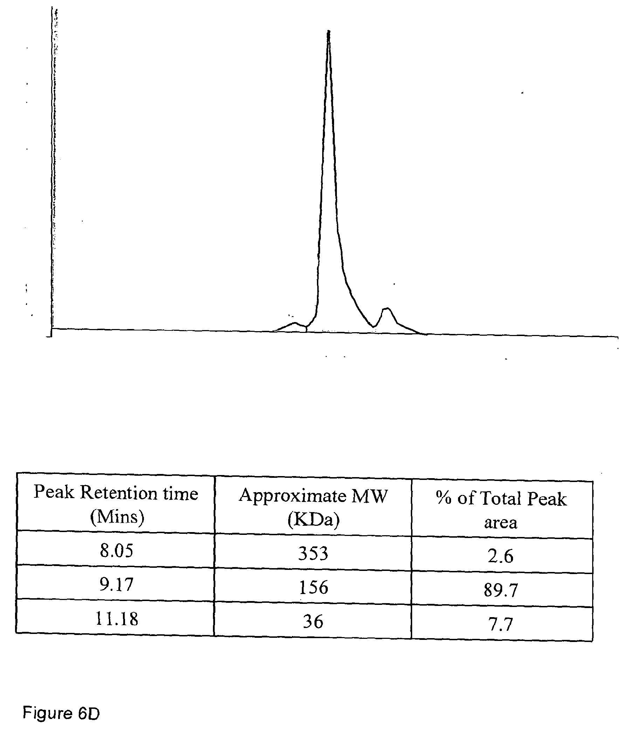

Figures 6A and 6B show the quantitative purification of anti-canine NGF antibody (HCA isotype) using a three-step method (Method I) comprising (1) anion exchange chromatography, (2) hydrophobic interaction chromatography and (3) size exclusion chromatography. Figure 6A shows the results of fractionation by size exclusion HPLC. Figure 6B shows a reducing SDS-PAGE gel of fractions following each step. Figure 6C and D show the quantitative purification of the anti-canine NGF antibodies described herein using a two-step method (Method II) comprising Captoadhere chromatography and anion exchange chromatography. Figure 6C shows SDS-PAGE analysis under non-reducing and reducing conditions. In Figure 6c, lane 1 is MWS, lane 2 is anti-canine NGF antibody 2µg/mL and 0µl reducing agent, lane 3 is anti-canine NGF antibody 4µg/mL and 0µl reducing agent, lane 4 is anti-canine NGF antibody 6µg/mL and 0µl reducing agent, lane 5 is MWS, lane 6 is anti-canine NGF antibody 2µg/mL and 3µl reducing agent, lane 7 is anti-canine NGF antibody 4µg/mL and 3µl reducing agent, lane 8 is anti-canine NGF antibody 6µg/mL and 3µl reducing agent and lane 9 is MWS. Figure 6D: size exclusion chromatography of the purified anti-canine NGF antibody.
Figure 7 shows a comparison of anti-canine NGF antibody (HCA isotype) purified by Methods I and II. Figure 7A: comparison by non-reducing and reducing SDS-PAGE. Figure 7B: comparison by anti-NGF ELISA.
Figure 8 shows body weight (upper panel) and temperature (lower panel) are stable following intravenous administration of anti-canine NGF antibodies (HCA isotype, purified by Method I) into dogs.
Figure 9 shows kinetic analysis of plasma anti-canine NGF monoclonal antibody concentration following intravenous injection to a dog. A beagle dog was injected intravenously with anti-NGF antibody at 2 mg/kg, samples of plasma were taken at the times indicated and anti-NGF monoclonal antibody was detected by NGF ELISA. The anti-canine NGF monoclonal antibody had a surprisingly long elimination (beta) phase half life of approximately 9 days.
Figure 10 shows that anti-canine NGF monoclonal antibodies (HCA isotype, purified by Method I) reduce inflammatory pain in dogs. Kaolin was injected into the footpad of beagle dogs at Day -1, antibody or vehicle control at Day 0 and lameness was measured by a visual scoring scale.

### Detailed description of the invention

Following extensive experimentation, the inventor has designed and constructed several canine monoclonal antibodies using different heavy chain constant domain isotypes and has surprisingly shown that useful properties can be deduced from their ability (or otherwise) to mediate downstream effector functions and in particular from their ability to bind to complement. Accordingly, the inventor has identified different biological effects mediated by different canine immunoglobulin subtypes. For the complement-binding canine antibody isotypes, this useful property is in directing cells bound by the same antibodies for complement directed cytotoxicity (CDC) *in-vivo.* An example where this functional property is desirable is in canine cancer therapy where antibodies directed to tumour antigens would then direct the complement system to target the cells which have been bound by the antibody, for destruction.

By contrast, antibody isotypes which do not bind complement and so do not cause CDC are preferred where complement activity is undesirable, for example in the proximity of nerves, in the eye or in already inflamed tissues, or simply due to the desire to reduce the risk of an unforeseen side effect of antibody. Clearly therefore, the ability to predict which canine isotypes are suitable for design and use of antibody therapies in canines is a highly desirable and useful.

Four different canine immunoglobulin G isotypes have been described (caIgG-A (canine immunoglobulin G isotype A), caIgG-B, caIgG-C, and caIgG-D - Tang et al., 2001). For simplicity (and to allow distinction of different antibody constructions and from light chain components) the heavy chain constant domains are termed HCA (caIgG-A), HCB (caIgG-B), HCC (caIgG-C) and HCD caIgG-D) herein.

### Purification of antibodies

A further surprising discovery was made by the inventor in the process of purifying the desirable HCA and HCD isoforms of anti-NGF antibodies in that neither bound to Protein A, the ligand used at manufacture-scale in industry in the form of Protein A affinity column chromatography to produce large scale purification of therapeutic proteins. Figure 5 shows the relative recovery of HCA, HCB, HCC and HCD isoforms of the anti-NGF antibodies by Protein A affinity chromatography at small scale. Consequently, other methods were needed to purify the HCA or HCD isoforms, as these could not be purified using Protein A affinity chromatography. After extensive experimentation, the inventor has surprisingly identified two alternative methods (referred to herein as Method I and Method II) which could be used to purify a caN-HCA-kLC antibody construct, or other canine antibodies which have the HCA or HCD heavy chain isotype.

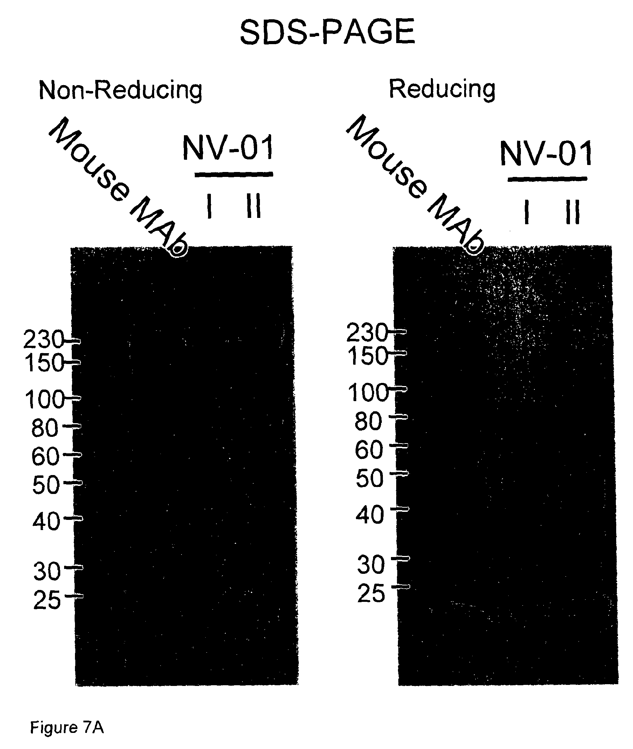

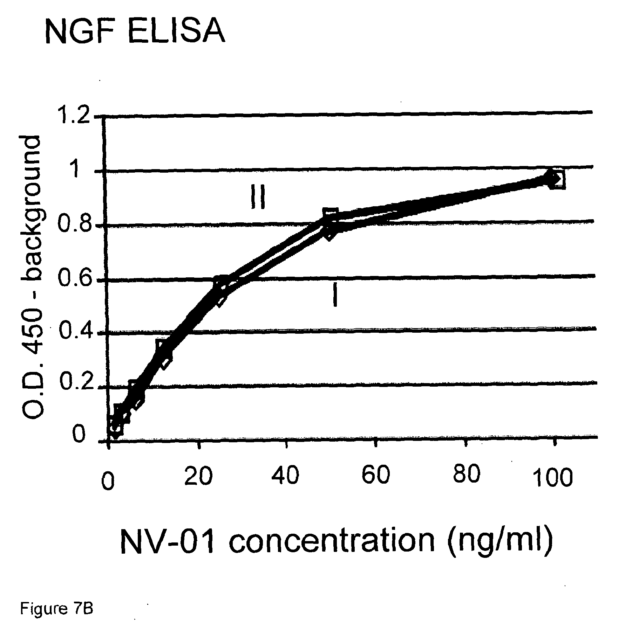

The first method comprises a combination of anion exchange chromatography, hydrophobic interaction chromatography and size exclusion chromatography. The second method comprises a combination of captoadhere affinity chromatography and anion exchange chromatography. Figure 6 illustrates the purification of the HCA containing anti-NGF antibody by each of the two methods. Highly purified material was obtained by each method and the two methods produced material with similar bioactivity by NGF ELISA (Figure 7). Since the HCA and HCD isotypes are more similarly related to one another than to HCB and HCC isotypes, the methods are used for both isotypes.

In a further exploration of antibody purification, the inventor surprisingly found that the aglycosyl HCB* isotype anti-NGF antibody, like the HCB isotype was still able to bind Protein A and so has the desirable property of lack of CDC activity and purification by Protein A chromatography.

### Canine safety testing

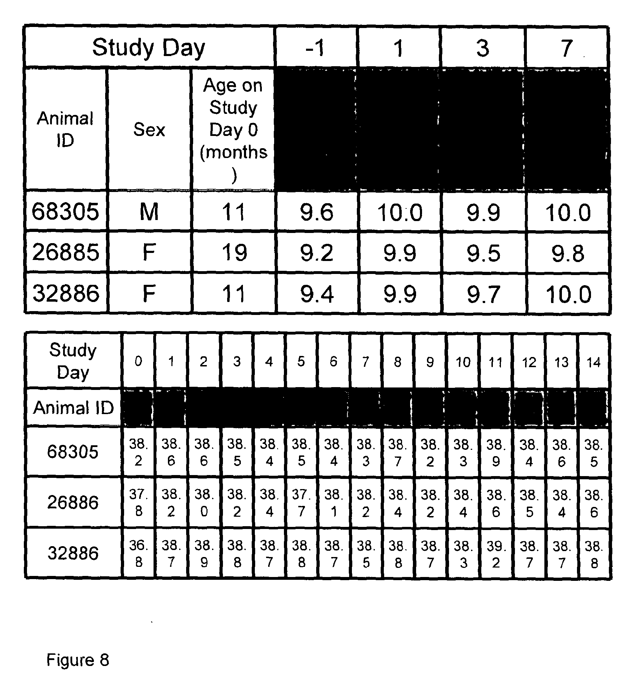

In order to demonstrate that the antibodies described herein, that are designed to have no unwanted CDC activity, are safe to give to dogs, the highly purified anti-NGF HCA isotype antibody was injected into three dogs by intravenous injection (following prior approval by the Institutional Animal Ethics Committee - CRL, Ireland). Figure 8 shows that in addition to a lack of behavioural changes observed by the veterinarians, the three dogs showed no weight change or pyrexia following injection of HCA isotype antibody (single 2 mg/kg dose).

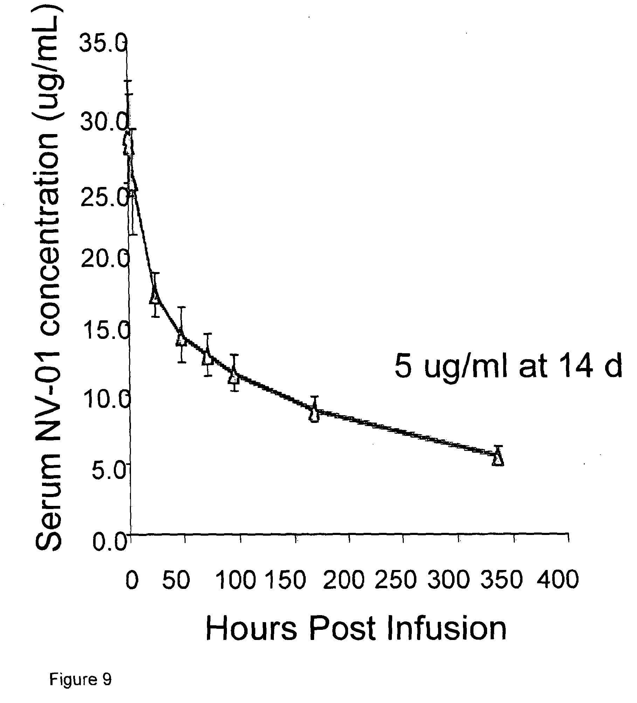

Plasma kinetics of the HCA isotype antibody in the three dogs was consistent with a two-phase distribution and clearance mechanism, including a long beta half-life of approximately 9 days (Figure 9). The lack of rapid clearance over the 14 days follow up period was consistent with their being no anti-antibody response to the canine antibody. By contrast human immunoglobulin heavy chain constant domains are immunogenic in dogs and they are cleared rapidly from the plasma at about 8 or 9 days post infusion (Richter 1999, Drug Met. Disp. 27, 21-25).

### Canine model of inflammation

All experiments were carried out with prior approval of the Institutional Ethics Committee (CRL, Ireland). Beagle dogs were injected (=day -1) with kaolin into the footpad of one hind leg in order to generate a self-resolving inflammation beginning approximately 24 hours later and which causes the dogs to become temporarily lame. In this model, once the initial inflammation response to kaolin recedes, the dogs become steadily less lame over the period of approximately 1-2 weeks and then make a full recovery.

Groups of 3 dogs were injected intravenously with either anti-canine (HCA isotype) NGF monoclonal antibodies at 200 µg/kg body weight or phosphate buffered saline as vehicle control (=day 0). The dogs were assessed for lameness over 7 days by a visual scoring method (score 0, no lameness (full weight bearing); score 1, slight lameness (not full weight bearing but walking well); score 2, moderate lameness (slightly weight bearing and not walking well), score 3, severe lameness (not weight bearing)). Observers were blinded to which dogs received which injection.

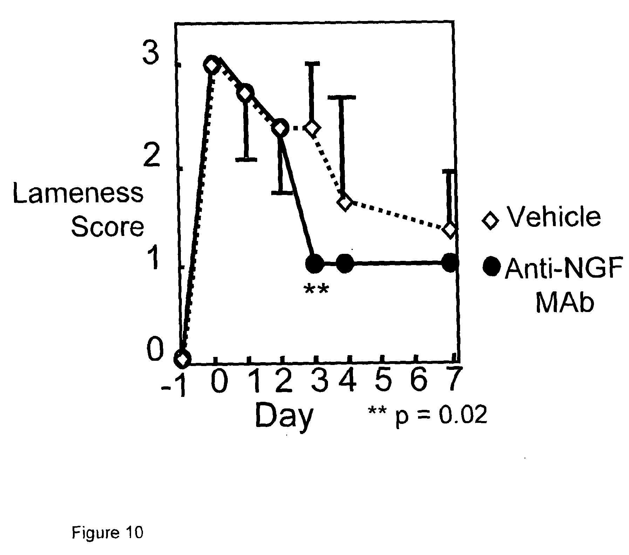

The results are shown in Figure 10. Lameness scores were reduced in the dogs receiving anti-NGF monoclonal antibodies by day 3 post-injection compared with vehicle control, indicating that the anti-NGF monoclonal antibodies had an effect in reducing the pain in the dogs over that seen with vehicle alone. The delayed activity is consistent with the plasma pharmacokinetics of anti-canine NGF monoclonal antibodies which demonstrated a slow tissue distribution (alpha) phase of approximately 30 hours and the relatively poor vascularisation of the footpad area. The results shown in Figure 10 show that the anti-canine NGF antibodies described herein reduce inflammatory pain in dogs with a consequent reduction in lameness.

Together the results described by this inventor demonstrate that purified canine antibodies constructed using the CDC inactive HCA isotype are safe and effective in canines and have a desirable long half-life.

### Definitions

Unless otherwise defined, all technical and scientific terms used herein have the meaning commonly understood by a person who is skilled in the art in the field of the present invention. The meaning and scope of the terms should be clear, however, in the event of any ambiguity, definitions provided herein take precedent over any dictionary or extrinsic definition.

Throughout the specification, unless the context demands otherwise, the terms "comprise" or "include", or variations such as "comprises" or "comprising", "includes" or "including" will be understood to imply the inclusion of a stated integer or group of integers, but not the exclusion of any other integer or group of integers.

As used herein, terms such as "a", "an" and "the" include singular and plural referents unless the context clearly demands otherwise. Thus, for example, reference to "an active agent" or "a pharmacologically active agent" includes a single active agent as well as two or more different active agents in combination, while references to "a carrier" includes mixtures of two or more carriers as well as a single carrier, and the like. Further, unless otherwise required by context, singular terms shall include pluralities and plural terms shall include the singular.

As herein defined, the term "pain" means an unpleasant sensory and emotional experience associated with actual or potential tissue damage, or described in terms of such damage.

In relation to operative or post-operative pain, the US Animal Welfare Act (Animal Welfare Act 2002. AWA regulations, CFR, Title 9 (Animals and Animal Products), Chapter 1 (Animal and Plant Health Inspection Service, Department of Agriculture). Subchapter A (Animal Welfare), Parts 1-4) defines a painful procedure as any procedure that would reasonably be expected to cause more than slight or momentary pain or distress in a human being to which that procedure was applied, that is, pain in excess of that caused by injections or other minor procedures. Therefore, if a canine undergoes a painful surgical procedure, the animal should receive postoperative analgesics.

In further instance, a canine may be experiencing significant or chronic pain as a result of an associated medical condition such as rheumatoid arthritis, osteoarthritis, inflammation or a cancerous or malignant condition.

The term "nociception" refers to the perception of noxious stimuli. As herein defined "neuropathic pain" (also known as 'neuralgia') is a pain that comes from problems with signals from the nerves. It may arise as a consequence of a lesion or disease affecting the somatosensory system. There are causes of neuropathic pain and it may be associated with abnormal sensations called dysesthesia, which occur spontaneously. Alternatively, it may be associated with allodynia which results when the pain comes on, or gets worse, with a touch or stimulus that would not normally cause pain. For example, a slight touch on the face may trigger pain if you have trigeminal neuralgia, or the pressure of the bedclothes may trigger pain if you have diabetic neuropathy. Neuropathic pain may also result from allodynia, where the pain comes on, or gets worse, with a touch or stimulus that would not normally cause pain. For example, a slight touch to the face may trigger pain if a subject has trigeminal neuralgia. Neuropathic pain relating to hyperalgesia means that severe pain results from a stimulus or touch that would normally cause only slight discomfort, while paraesthesia means that uncomfortable or painful feelings occur even when there is nothing in contact with the area causing the pain, for example pins and needles. Other forms of neuropathic pain involve pruritis or itch, which can be associated with allergic or inflammatory responses in the skin and inflammatory pain resulting from tissue damage and repair processes

As defined herein, the term "NGF neutralising antibody" or similar describes an antibody that is capable of neutralising the biological activation and signalling of NGF. The neutralising antibody, which may also be referred to as an antagonistic antibody, or a blocking antibody, specifically, and preferably selectively, binds to NGF and inhibits one or more biological activities of NGF. For example, the neutralising antibody may inhibit the binding of a NGF to its target ligand, such as the cell membrane bound TrkA or p75 receptors.

As used herein, the term "biological activity" refers to any one or more inherent biological properties of a molecule (whether present naturally as found in vivo, or provided or enabled by recombinant means). Biological properties include but are not limited to receptor binding and/or activation; induction of cell signalling or cell proliferation, inhibiting cell growth, induction of cytokine or chemokine production, induction of apoptosis, and enzymatic activity.

The term "complementarity determining region (CDR)", as used herein, refers to amino acid sequences which together define the binding affinity and specificity of the natural Fv region of a native immunoglobulin binding site as delineated by Kabat et al. (Kabat, E.A., Wu, T.T., Perry, H., Gottesman, K. and Foeller, C. (1991) Sequences of Proteins of Immunological Interest, Fifth Edition. NIH Publication No. 91-3242). The term "framework region (FR)", as used herein, refers to amino acid sequences interposed between CDRs. These portions of the antibody serve to hold the CDRs in appropriate orientation (allows for CDRs to bind antigen).

The term "constant region (CR)" as used herein, refers to the portion of the antibody molecule which confers effector functions. In the present invention, constant regions typically mean canine constant regions, that is that the constant regions of the subject canininsed antibodies are derived from canine immunoglobulins.

The term "chimeric antibody" as used herein refers to an antibody containing sequences derived from two different antibodies, which typically are of different species. Most typically chimeric antibodies comprise variable domains derived from a donor specifies which bind specifically to a target epitope and constant domains derived from antibodies obtained from the target species to whom the antibody is to be administered.

The term "immunogenicity" as used herein refers to a measure of the ability of a targeting protein or therapeutic moiety to elicit an immune response (humoral or cellular) when administered to a recipient. The present invention is concerned with the immunogenicity of the subject caninised antibodies. Preferably the antibodies described herein have no immunogenicity, that is that no neutralising antibodies will be raised against them when administered to a canine, and further, no effector functions are mediated by the Fc regions of the antibody.

The term "identity" or "sequence identity" as used herein, means that at any particular amino acid residue position in an aligned sequence, the amino acid residue is identical between the aligned sequences. The term "similarity" or "sequence similarity" as used herein, indicates that, at any particular position in the aligned sequences, the amino acid residue is of a similar type between the sequences. For example, leucine may be substituted for an isoleucine or valine residue. This may be referred to as conservative substitution. Preferably when the amino acid sequences disclosed herein are modified by way of conservative substitution of any of the amino acid residues contained therein, these changes have no effect on the binding specificity or functional activity of the resulting antibody when compared to the unmodified antibody.

Sequence identity with respect to a (native) polypeptide as described herein and its functional derivative relates to the percentage of amino acid residues in the candidate sequence which are identical with the residues of the corresponding native polypeptide, after aligning the sequences and introducing gaps, if necessary, to achieve the maximum percentage homology, and not considering any conservative substitutions as part of the sequence identity. Neither N- or C-terminal extensions, nor insertions shall be construed as reducing sequence identity or homology. Methods and computer programs for performing an alignment of two or more amino acid sequences and determining their sequence identity or homology are well known to the person skilled in the art. For example, the percentage of identity or similarity of 2 amino acid sequences can be readily calculated using algorithms e.g. BLAST (Altschul et al. 1990), FASTA (Pearson & Lipman 1988), or the Smith-Waterman algorithm (Smith & Waterman 1981).

As used herein, reference to an amino acid residue having the "highest homology" to a second amino acid residue refers to the amino acid residue which has the most characteristics or properties in common with the second amino acid residue. In determining whether an amino acid residue has the highest homology to a second amino acid residue, an assessment may typically be made of factors such as, but not limited to, charge, polarity, hydrophobicity, side arm mass and side arm dimension.

The term "corresponding position" as used herein to refer to an amino acid residue that is present in a second sequence at a position corresponding to a specified amino acid residue in a first sequence is intended to refer to the position in the second sequence which is the same position as the position in the first sequence when the two sequences are aligned to allow for maximum sequence identity between the two sequences. Amino acid residues at corresponding positions have the same Kabat numbering.

The term "consists essentially of" or "consisting essentially of" as used herein means that a polypeptide may have additional features or elements beyond those described provided that such additional features or elements do not materially affect the ability of the antibody or antibody fragment to have binding specificity to canine NGF. That is, the antibody or antibody fragments comprising the polypeptides may have additional features or elements that do not interfere with the ability of the antibody or antibody fragments to bind to canine NGF and antagonise canine NGF functional activity. Such modifications may be introduced into the amino acid sequence in order to reduce the immunogenicity of the antibody. For example, a polypeptide consisting essentially of a specified sequence may contain one, two, three, four, five or more additional, deleted or substituted amino acids, at either end or at both ends of the sequence provided that these amino acids do not interfere with, inhibit, block or interrupt the role of the antibody or fragment in binding to canine NGF and sequestering its biological function. Similarly, a polypeptide molecule which contributes to the canine NGF antagonistic antibodies described herein may be chemically modified with one or more functional groups provided that such functional groups do not interfere with the ability of the antibody or antibody fragment to bind to canine NGF and antagonise its function.

As used herein, the term "effective amount" or "therapeutically effective amount" means the amount of an agent, binding compound, small molecule, fusion protein or peptidomimetic described herein which is required to deliver the required therapeutic effect.

The terms "polypeptide", "peptide", or "protein" are used interchangeably herein to designate a linear series of amino acid residues connected one to the other by peptide bonds between the alpha-amino and carboxy groups of adjacent residues. The amino acid residues are usually in the natural "L" isomeric form. However, residues in the "D" isomeric form can be substituted for any L-amino acid residue, as long as the desired functional property is retained by the polypeptide.

As herein defined an "antibody" encompasses antigen-binding proteins which specifically bind to a target antigen of interest, in this case canine nerve growth factor, having one or more polypeptides that can be recombinantly prepared or which are genetically encodable by immunoglobulin genes, or fragments of immunoglobulin genes. The term "antibody" encompasses monoclonal and chimeric antibodies, in particular caninised antibodies, and further encompasses polyclonal antibodies or antibodies of any class or subtype. An "antibody" further extends to hybrid antibodies, bispecific antibodies, heteroantibodies and to functional fragments thereof which retain antigen binding.

The phrase "specifically binds to" refers to the binding of an antibody to a specific protein or target which is present amongst a heterogeneous population of proteins. Hence, when present in specific immunoassay conditions, the antibodies bind to a particular protein, in this case canine NGF, and do not bind in a significant amount to other proteins present in the sample.

As defined herein, a "canine" may also be referred to as a "dog". Canines can be categorised as belonging to the subspecies with the trinomial name Canis lupus familiaris (*Canis familiaris domesticus)* or Canis lupus dingo. Canines include any species of dog and includes both feral and pet varieties, the latter also being referred to as companion animals.

The present invention will now be described with reference to the following examples which are provided for the purpose of illustration and are not intended to be construed as being limiting on the present invention. The methods and techniques of the present invention are generally performed according to conventional methods well known in the art and as described in various general and more specific references that are cited and discussed throughout the present specification unless otherwise indicated.

### EXAMPLES

### Example 1 - Design and production of anti-canine NGF antibodies having different canine isotypes

Antibodies directed to canine NGF (termed caN antibodies) were designed and constructed using identical variable heavy domains (VH) joined to heavy chain constant domains (CH2 and CH3) selected from HCA (SEQ ID NO:1), HCB (SEQ ID NO:2), HCC (SEQ ID NO:3) or HCD (SEQ ID NO:4). A variable light chain (VL) was joined to the canine kappa constant domain (SEQ ID NO:5). The combined amino acid sequences were converted to expressible form in mammalian cells by the optimal selection of codons and full chemical gene synthesis and cloning into a mammalian cell expression vector pcDNA3.1+. Specifically, the designed amino acid sequences were constructed into synthetic cDNA-expressible form and cloned into a mammalian cell expression vector pcDNA3.1(+). Whole antibody sequences were produced by combining caninised variable domain sequences with C-terminal canine constant heavy or constant light chain sequences. The caninised aD11 VH domain was combined with each of the four heavy chain isotypes HCA, HCB, HCC and HCD (SEQ ID NO:1- SEQ ID NO:4) and the caninised aD11 VL domain with the canine kappa light chain constant domain (SEQ ID NO:5). The combined amino acid sequences were converted to expressible form in mammalian cells by the optimal selection of codons and full chemical gene synthesis and cloning into a mammalian cell expression vector pcDNA3.1+.

Combinations of caninised heavy and light chain cDNA plasmids (caN-HCA-kLC using SEQ ID NO:5 plus SEQ ID NO:1 (HCA); caN-HCB-kLC using SEQ ID NO:5 plus SEQ ID NO:2 (HCB); caN-HCC-kLC using SEQ ID NO:5 plus SEQ ID NO:3 (HCC) and caN-HCD-kLC using SEQ ID NO:5 plus SEQ ID NO:4 (HCD)) were transfected into CHO cells, the supernatants harvested and reacted in ELISA format with NGF. Following incubation and wash steps, the bound canine antibody was detected by reactivity with a goat-anti canine IgG specific polyclonal antibody linked to horseradish peroxidase (HRP) and developed using TMB. The optical density of the resulting product was measured at 450nm and compared with that from mock empty vector transfected supernatant. The results of binding to NGF for the 4 caninised antibody isotypes are shown in Figure 1. Each of these antibodies has the same light chain (caN-kLC), this being a light chain comprising a canine kappa constant domain. Each antibody has a different heavy chain constant domain. Accordingly a specific heavy chain variable domain is combined with one of 4 different constant domains (caN-HCA, caN-HCB, caN-HCC or caN-HCD). Equivalent binding to NGF was observed for each of the canine heavy chain isotypes.

Antibody supernatants were tested for NGF binding by ELISA assay (Figure 1A) and NGF neutralisation by TF-1 cell proliferation inhibition assay (Figure 1B). As can be seen in Figure 1, the four isotypes had equivalent activity to one another in these assays, that is, they all bound specifically to canine NGF.

### Example 2: Complement deposition induced by NGF-captured caninised antibodies

The four antibody-containing supernatants were then assessed for their ability to bind complement when bound to NGF using a complement C1q ELISA. Plates were coated with 100µl/well of 5µg/ml mouse NGF and blocked with 5% BSA/PBS. Coated wells were incubated for 1 hour at room temperature with cell culture supernatants, containing recombinant caninised anti-NGF IgG isotypes, diluted in PBS/1% BSA (100µl/well). The plates were washed and incubated for 1 hour at room temperature with 100µl/well of human serum diluted 1/100 in veronal buffered saline containing 0.5mM MgCl₂, 2mM CaCl₂, 0.05% Tween-20, 0.1% gelatin and 0.5% BSA. After washing, plates were incubated with 100 µl of a 1/800 dilution of sheep anti-C1q-HRP (Serotec) in PBS/1% BSA. After washing, plates were developed by the addition of 100µl TMB substrate. All complement C1q binding expressed as A450 minus heat-inactivated complement background. Development was stopped by the addition of 100µl of 2N H₂SO₄ and absorbance read at 450 nm.

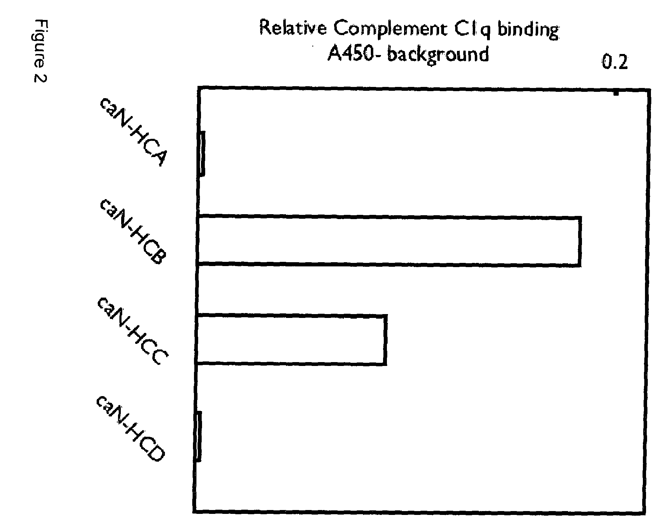

The results are shown in Figure 2. These results show binding of C1q to immobilised caninised HCB and HCC type antibodies and no binding of C1q to caninised HCA and HCD type antibodies. Hence, the results surprisingly indicate that different canine derived heavy chains exhibit different complement binding and hence activation characteristics and that the caninised antibodies with type HCA and HCD heavy chains unexpectedly are preferable for use in antagonising canine NGF. Accordingly, the ability to produce an antibody which binds specifically to canine NGF, yet which does not mediate CDC is highly advantageous, as an antibody which binds specifically to NGF, yet which mediated an immune response in proximity to the cells expressing NGF, would be highly undesirable.

### Example 3: Complement binding of NGF captured N-glycosylated and aglycosyl variants of anti-canine-NGF monoclonal antibodies with HCB and HCC heavy chain isotypes.

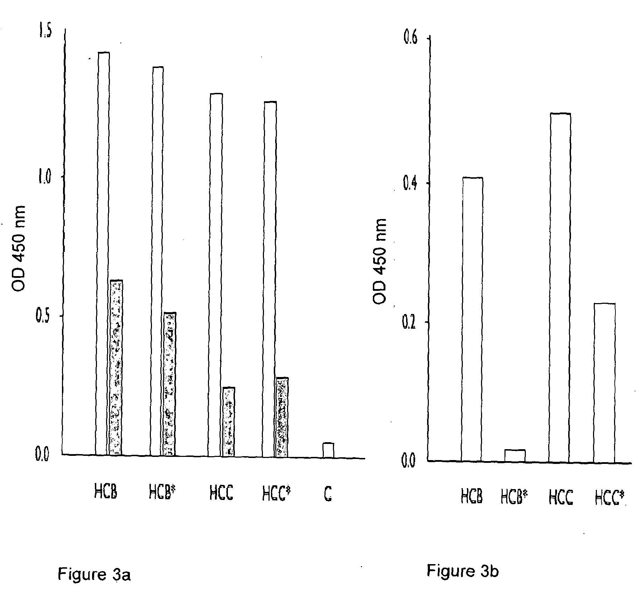

A comparison of the binding of N-glycosylated and aglycosyl variants of anti-canine-NGF monoclonal antibodies to NGF with HCB and HCC heavy chain isotypes was carried out. Expression vectors encoding the light and heavy chain pairs described by SEQ ID NO:5 and SEQ ID NO:2 (HCB), SEQ ID NO:5 and SEQ ID NO:6 (HCB*), SEQ ID NO:5 and SEQ ID NO:3 (HCC), or SEQ ID NO:5 and SEQ ID NO:7 (HCC*) were co-transfected into CHO cells and the supernatants compared by binding ELISA to mouse NGF. The results are shown in Figure 3. The left hand panel shows detection by ELISA of expression of anti-NGF MAbs constructed with HCB heavy chain (HCB), aglycosyl HCB heavy chain (HCB*), HCC heavy chain (HCC) or aglycosyl HCC heavy chain (HCC*) - the open bars show undiluted supernatant, the shaded bars 1/10 diluted supernatant and C shows an undiluted negative control supernatant. Equivalent binding to NGF was observed.

Similarly, antibodies designed to remove the constant domain N-linked glycosylation site of HCB and HCC (referred to as HCB* (SEQ ID NO: 6) and HCC* (SEQ ID NO:7)) were co-expressed with light chain and assessed for complement activity (Figure 3) in an attempt to ablate their complement binding. Surprisingly, the aglycosylated HCB* was not capable of binding complement, however the aglycosylated HCC* remained capable of binding complement. The identification of canine derived glycosylated and aglycosylated heavy chains which do not mediate complement fixing is a particularly advantageous finding as NGF is a soluble mediator involved in nociception.

CHO cell transfectant supernatants from were tested for their ability to recruit complement using the C1q ELISA assay described in Example 2. The results are shown in Figure 3- right hand panel. Together the results in Figure 3 demonstrate that the ability to recruit complement C1q was abolished by removal of the N-linked glycosylation site in the B type heavy chain (HCB*) and was diminished by a similar mutation in the C type heavy chain (HCC*).

Accordingly, it is shown herein, quite surprisingly, that where an antibody has a canine-derived heavy chain of the HCA, HCD subtype or aglycosylated HCB* isotype, the binding of the antibody to canine NGF does not result in complement activation (and potentially other downstream effector functions, such as ADCC and ADCP). Hence, said antibodies, in antagonising the biological functional activity of a target, such as canine NGF, by preventing binding of canine NGF to cell membrane bound TrkA or p75 receptors, inhibit the associated downstream intracellular signalling cascade. Furthermore, as NGF expression frequently occurs in the proximity of nerves, such NGF antagonising or neutralising antibodies, which have canine derived heavy chain of the HCA, HCD or HCB* subtype, sequester canine NGF biological activity without recruiting a wider immune response. Hence, the results surprisingly indicate that different canine derived heavy chains exhibit different complement binding and activation characteristics and that the caninised antibodies with type HCA and HCD heavy chains have been unexpectedly shown to be preferable for use in antagonising canine NGF. The identification of canine derived heavy chains which do not mediate complement fixing is a particularly advantageous finding as NGF is a soluble mediator. Such functional properties are both unexpected and highly desirable.

### Example 4: Production of antibodies with canine heavy chain constant domains to other antigens: VEGF and CD20 and their binding to complement.

Given the surprising result that different canine heavy chain isotypes have differential binding to complement, anti-VEGF and anti-CD20 antibodies were similarly constructed using canine heavy chain constant domains expressed in CHO cells using the same methodology as described in Example 1. Assay results from these antibody supernatants in the complement ELISA are shown in Figure 4. The results compared to the anti-NGF antibodies described above for their ability to recruit complement following binding to their cognate antigen.

The results are shown in Figure 4. Panel A shows that caninised anti-NGF MAb constructed using canine heavy chain isotypes B and C and humanised antibodies constructed using human heavy chain isotypes IgG1 and IgG4 were captured onto NGF coated plates, incubated with human serum and bound C1q was detected by ELISA using anti-C1q polyclonal antibodies conjugated to HRP. Panel B shows the results of complement C1q binding to VEGF-captured caninised anti-VEGF MAbs constructed using canine heavy chain isotypes A and B (SEQ ID NO:8, SEQ ID NO:9). Panel C shows the results of complement C1q binding to anti-CD20 MAb captured on huCD20 extracellular domain peptide (muB-2a: murine anti-human CD20 MAb and muB-HCB: murine anti-human CD20 MAb expressed as a chimeric fusion protein with canine heavy chain isotype B (SEQ ID NO:9). All complement C1q binding expressed as A450 minus heat-inactivated complement background.

Together these data show that anti-VEGF antibodies constructed using HCB but not HCA canine heavy chain constant domains could recruit complement and an anti-CD20 antibody constructed using canine HCB could bind complement and so parallel the differential binding of anti-NGF antibody isotypes to complement described above. In summary, these data support the surprising observation that antigen-captured antibodies constructed with HCB and HCC isotypes bind complement whereas HCA and HCD do not. The identification that HCB and HCC isotypes bind complement is particularly advantageous for tumour cell killing e.g. of VEGF-expressing or CD20 expressing tumour cells.

The results of these experiments support the unexpected finding from Example 1 that antibodies comprising canine heavy chain constant domain HCA, HCD and aglycosylated HCB (HCB*) isotypes result in immunoglobulins which do not mediate CDC activity. Accordingly, the inventor has identified for the first time that such canine derived heavy chains have utility in immunoglobulins for use in therapeutic methods where a CDC mediated immune response is not desired. Examples of such uses may be found in the inhibition by canine immunoglobulins of cytokines or chemokines, growth factors, hormones and other extracellular mediators including complement itself *in vivo* or in diseases such as pain, macular degeneration or inflammation.

While the HCA, HCD and HCB* isotypes are most useful for design of CDC inactive antibodies, the inventor has also surprisingly identified that canine antibodies of HCB (caIgG-B) and HCC (caIgG-C) isotypes are useful in the design of CDC active antibodies. Such antibodies are useful when targeting cells for destruction, for example in cancer therapy. There are many human tumour antigens that are targeted using CDC active antibodies, including CD20, HER2 and the EGFR so canine antibodies constructed using HCB and HCC isotypes will have parallel uses in canines.

### Example 5: Purification of anti-NGF monoclonal antibodies following expression in CHO cells

Since canine anti-NGF monoclonal antibodies of the HCA and HCD isotypes have desirable lack of binding to complement (Figure 2), but bind weakly to Staphylococcus Protein A (Figure 5), alternative methods of purification were developed (Figure 6). Anti-canine NGF monoclonal antibodies (constructed using heavy chain isotype HCA) were expressed in CHO cells and following extensive experimentation it was surprisingly found that the canine anti-NGF antibody could be fractionated to high purity (>89% monomeric IgG peak, as shown in Fig 6A, 6D) by two alternative purification methods.

In the first method, anti-canine NGF monoclonal antibody was purified by anion exchange chromatography, hydrophobic interaction chromatography and size exclusion chromatography (Method I - Figure 6A and B). In the second method, the anti-NGF antibody could be purified by Captoadhere affinity chromatography followed by anion exchange chromatography (Method II - Figure 6C and D).

The main peak of anti-NGF monoclonal antibody purified by either method corresponds to a molecular weight of approximately 150 kDa. Comparison by SDS-PAGE and ELISA (Figure 7) illustrates that Methods I and II produce antibody preparations with similar purity and bioactivity. Purified anti-NGF monoclonal antibodies produced by these methods were tested in the TF-1 NGF neutralisation assay (described in Figure 1) and shown to have high potency (IC50 13 pM anti-NGF neutralised 37 pM NGF; not shown).

### Example 6: Anti-canine NGF monoclonal antibodies can be safely administered intravenously to canines and do not cause pyrexia

Anti-canine NGF monoclonal antibodies derived from expression vectors containing canine HCA type heavy chain were expressed in CHO cells and purified by a combination of ion exchange chromatography, hydrophobic interaction chromatography and size exclusion chromatography (Method I, Figure 6A and B) and buffer exchanged into phosphate buffered saline. The antibodies were injected intravenously into beagle dogs at 2mg/kg body weight and assessed for signs of toxicity by visual inspection by a veterinarian, change in body weight, body temperature and plasma biochemistry. Figure 8 illustrates the body weight and temperature measurements. No changes were observed in these or any plasma biochemistry analyte measured (including sodium, potassium, chloride, calcium, phosphate, urea, creatinine, glucose, cholesterol, bilirubin, alanine transaminase, alkaline phosphatase, amylase, lipase, total protein or albumin: not shown).

### Example 7: Plasma pharmacokinetics of anti-canine (HCA isotype) NGF monoclonal antibodies in-vivo demonstrates long serum half-life and lack of immunogenicity

Anti-canine NGF monoclonal antibodies derived from expression vectors expressing canine HCA type heavy chain were expressed in CHO cells and purified by a combination of ion exchange chromatography, hydrophobic interaction chromatography and size exclusion chromatography and buffer exchanged into phosphate buffered saline (Method 1, Figure 6A and B). The antibodies were injected intravenously into beagle dogs at 2mg/kg body weight and plasma samples were taken at various times over the following 2 weeks. Diluted plasma samples were assessed for anti-canine NGF antibody concentration by ELISA using NGF as target and anti-canine polyclonal antibody-horseradish peroxidase secondary reagent and developed as per Figure 1. The results are shown in Figure 9. The plasma concentrations measured were consistent with two-phase kinetics, with a tissue distribution (alpha) phase half-life of approximately 33 hours and surprisingly long elimination (beta) phase of approximately 9 days.

The absence of a sharp decline in plasma concentration of anti-canine NGF antibody concentration between 100 and 300 hours demonstrates that there are neither pre-existing neutralising antibodies to recombinant anti-NGF monoclonal antibodies in dog blood, nor were any such neutralising antibodies generated following infusion. By comparison, recombinant human immunoglobulin based proteins are neutralised by antibodies in dog blood at approximately 200 hours post infusion (Richter et al, Drug Metabolism and Disposition 27: 21, 1998). These results therefore show that anti-canine NGF antibodies described herein have a long serum half-life (approximately 9 days) in vivo following intravenous injection and that there are neither pre-existing antibodies nor newly generated antibodies that neutralise the injected anti-NGF antibodies over time.

### Example 8: Effect of anti-canine NGF monoclonal antibodies in reducing inflammatory pain in-vivo

### Antibody therapy:

Anti-canine NGF monoclonal antibodies derived from expression vectors including canine HCA type heavy chain were expressed in CHO cells and purified by a combination of ion exchange chromatography, hydrophobic interaction chromatography and size exclusion chromatography (Method I) and buffer exchanged into phosphate buffered saline.

### Canine model of inflammation:

All experiments were carried out with prior approval of the Institutional Ethics Committee (CRL, Ireland). Beagle dogs were injected (= day -1) with kaolin into the footpad of one hind leg in order to generate a self-resolving inflammation beginning approximately 24 hours later and which causes the dogs to become temporarily lame. In this model, once the initial inflammation response to kaolin recedes, the dogs become steadily less lame over the period of approximately 1-2 weeks and then make a full recovery.

Groups of 3 dogs were injected intravenously with either anti-canine NGF monoclonal antibodies at 200µg/kg body weight or phosphate buffered saline as vehicle control (=day 0). The dogs were assessed for lameness over 7 days by a visual scoring method (score 0, no lameness (full weight bearing); score 1, slight lameness (not full weight bearing but walking well); score 2, moderate lameness (slightly weight bearing and not walking well), score 3, severe lameness (not weight bearing)). Observers were blinded to which dogs received which injection.

The results are shown in Figure 10. Lameness scores were reduced in the dogs receiving anti-NGF monoclonal antibodies by day 3 post-injection compared with vehicle control, indicating that the anti-NGF monoclonal antibodies had an effect in reducing the pain in the dogs over that seen with vehicle alone. The delayed activity is consistent with the plasma pharmacokinetics of anti-canine NGF monoclonal antibodies which demonstrated a slow tissue distribution (alpha) phase of approximately 30 hours and the relatively poor vascularisation of the footpad area. The results shown in Figure 10 show that the anti-canine NGF antibodies described herein reduce inflammatory pain in dogs with a consequent reduction in lameness.

## Claims

1. An antibody, fusion protein or a binding fragment thereof for use in the therapeutic treatment of a canine where target neutralisation is desired in the absence of undesirable effector function, wherein said antibody, fusion protein or binding fragment has a heavy chain constant domain comprising the amino acid sequence of SEQ ID NO:8, SEQ ID NO:11 or SEQ ID NO:13, wherein the amino acid sequence of the heavy chain minimises the activation of downstream immune system effector functions when the antibody, fusion protein or binding fragment is bound to its target antigen.

2. The antibody, fusion protein or binding fragment for use as claimed in claim 1 wherein the therapeutic treatment of the canine relates to the treatment, inhibition or amelioration of pain or inflammation in the canine.

3. The antibody, fusion protein or binding fragment for use as claimed in claim 1 or claim 2 wherein the therapeutic treatment of the canine relates to the treatment of arthritis or an arthritic condition.

4. The antibody, fusion protein or binding fragment for use as claimed in claim 2 wherein the pain is selected from the group consisting of neuropathic pain, oncologic pain, pain associated with, or resulting from, rheumatoid arthritis, pain associated with, or resulting from, osteoarthritis, pain associated with, or resulting from, inflammation and pain associated with, or resulting from, pruritis.

5. The antibody, fusion protein or binding fragment for use as claimed in any one of claims 1 to 4 wherein the downstream immune system effector functions are selected from the group consisting of complement dependent cytotoxicity, antibody dependent cell mediated cytotoxicity and antibody dependent cellular pathogenesis.

6. The antibody, fusion protein or binding fragment for use as claimed in claim 5 wherein the antibody has a heavy chain comprising the amino acid sequence of SEQ ID NO:6.

7. The antibody, fusion protein or binding fragment for use as claimed in any one of claims 1 to 6 wherein the target antigen is selected from the group consisting of a cytokine, a chemokine, a growth factor, a cell surface receptor, a virus and a component of the complement cascade.

8. An antibody or a fusion protein or a binding fragment thereof for use in the therapeutic treatment of a canine where target destruction is desired, wherein said antibody, fusion protein or binding fragment has a heavy chain constant domain comprising the amino acid sequence of SEQ ID NO:9, SEQ ID NO:10, or SEQ ID NO:14 wherein the amino acid sequence of the heavy chain mediates the activation of downstream immune system effector functions when the antibody, fusion protein or binding fragment is bound to its target antigen.

9. The antibody, fusion protein or binding fragment for use as claimed in claim 8 wherein the therapeutic treatment relates to the treatment of cancer or infectious disease in the canine.

10. The antibody, fusion protein or binding fragment for use as claimed in claim 9 wherein the downstream immune system effector functions are selected from the group consisting of complement dependent cytotoxicity, antibody dependent cell mediated cytotoxicity and antibody dependent cellular pathogenesis.

11. The antibody, fusion protein or binding fragment for use as claimed in claim 9 or claim 10 wherein the target antigen is a cancer specific antigen.

12. The antibody, fusion protein or binding fragment for use as claimed in claim 11 wherein the cancer specific antigen is selected from the group consisting of a cytokine, a chemokine, a growth factor, a cell surface receptor, a virus and a component of the complement cascade.

13. The antibody, fusion protein or binding fragment for use as claimed in claim 11 wherein the cancer specific antigen is selected from the group consisting of proteins CD2, CD4, CD8, CD20, EGFR, VEGFR and HER2.

## Patentansprüche

1. Ein Antikörper, ein Fusionsprotein oder ein Bindungsfragment davon zur Verwendung bei der therapeutischen Behandlung eines Hundes, bei der eine Zielneutralisierung in Abwesenheit einer unerwünschten Effektorfunktion erwünscht ist, wobei der Antikörper, das Fusionsprotein oder das Bindungsfragment eine konstante Domäne der schweren Kette aufweist, die die Aminosäuresequenz von SEQ ID NO: 8, SEQ ID NO: 11 oder SEQ ID NO: 13 beinhaltet, wobei die Aminosäuresequenz der schweren Kette die Aktivierung von nachgeschalteten Effektorfunktionen des Immunsystems minimiert, wenn der Antikörper, das Fusionsprotein oder das Bindungsfragment an sein Zielantigen gebunden ist.

2. Antikörper, Fusionsprotein oder Bindungsfragment zur Verwendung gemäß Anspruch 1, wobei sich die therapeutische Behandlung des Hundes auf die Behandlung, Inhibierung oder Verbesserung von Schmerz oder Entzündung bei dem Hund bezieht.

3. Antikörper, Fusionsprotein oder Bindungsfragment zur Verwendung gemäß Anspruch 1 oder Anspruch 2, wobei sich die therapeutische Behandlung des Hundes auf die Behandlung von Arthritis oder einem arthritischen Leiden bezieht.

4. Antikörper, Fusionsprotein oder Bindungsfragment zur Verwendung gemäß Anspruch 2, wobei der Schmerz aus der Gruppe ausgewählt ist, die aus Folgendem besteht: neuropathischem Schmerz, onkologischem Schmerz, mit rheumatoider Arthritis assoziiertem oder daraus resultierendem Schmerz, mit Osteoarthrose assoziiertem oder daraus resultierendem Schmerz, mit Entzündung assoziiertem oder daraus resultierendem Schmerz und mit Pruritus assoziiertem oder daraus resultierendem Schmerz.

5. Antikörper, Fusionsprotein oder Bindungsfragment zur Verwendung gemäß einem der Ansprüche 1 bis 4, wobei die nachgeschalteten Effektorfunktionen des Immunsystems aus der Gruppe ausgewählt sind, die aus Folgendem besteht: komplementabhängiger Zytotoxizität, antikörperabhängiger zellvermittelter Zytotoxizität und antikörperabhängiger zellulärer Pathogenese.

6. Antikörper, Fusionsprotein oder Bindungsfragment zur Verwendung gemäß Anspruch 5, wobei der Antikörper eine schwere Kette aufweist, die die Aminosäuresequenz von SEQ ID NO: 6 beinhaltet.

7. Antikörper, Fusionsprotein oder Bindungsfragment zur Verwendung gemäß einem der Ansprüche 1 bis 6, wobei das Zielantigen aus der Gruppe ausgewählt ist, die aus Folgendem besteht: einem Zytokin, einem Chemokin, einem Wachstumsfaktor, einem Zelloberflächenrezeptor, einem Virus und einer Komponente der Komplementkaskade.

8. Ein Antikörper oder ein Fusionsprotein oder ein Bindungsfragment davon zur Verwendung bei der therapeutischen Behandlung eines Hundes, bei der eine Zielzerstörung erwünscht ist, wobei der Antikörper, das Fusionsprotein oder das Bindungsfragment eine konstante Domäne der schweren Kette aufweist, die die Aminosäuresequenz von SEQ ID NO: 9, SEQ ID NO: 10 oder SEQ ID NO: 14 beinhaltet, wobei die Aminosäuresequenz der schweren Kette die Aktivierung von nachgeschalteten Effektorfunktionen des Immunsystems vermittelt, wenn der Antikörper, das Fusionsprotein oder das Bindungsfragment an sein Zielantigen gebunden ist.

9. Antikörper, Fusionsprotein oder Bindungsfragment zur Verwendung gemäß Anspruch 8, wobei sich die therapeutische Behandlung auf die Behandlung von Krebs oder einer Infektionskrankheit des Hundes bezieht.

10. Antikörper, Fusionsprotein oder Bindungsfragment zur Verwendung gemäß Anspruch 9, wobei die nachgeschalteten Effektorfunktionen des Immunsystems aus der Gruppe ausgewählt sind, die aus Folgendem besteht: komplementabhängiger Zytotoxizität, antikörperabhängiger zellvermittelter Zytotoxizität und antikörperabhängiger zellulärer Pathogenese.

11. Antikörper, Fusionsprotein oder Bindungsfragment zur Verwendung gemäß Anspruch 9 oder Anspruch 10, wobei das Zielantigen ein krebsspezifisches Antigen ist.

12. Antikörper, Fusionsprotein oder Bindungsfragment zur Verwendung gemäß Anspruch 11, wobei das krebsspezifische Antigen aus der Gruppe ausgewählt ist, die aus Folgendem besteht: einem Zytokin, einem Chemokin, einem Wachstumsfaktor, einem Zelloberflächenrezeptor, einem Virus und einer Komponente der Komplementkaskade.

13. Antikörper, Fusionsprotein oder Bindungsfragment zur Verwendung gemäß Anspruch 11, wobei das krebsspezifische Antigen aus der Gruppe ausgewählt ist, die aus den Proteinen CD2, CD4, CD8, CD20, EGFR, VEGFR und HER2 besteht.

## Revendications

1. Un anticorps, une protéine de fusion ou un fragment de liaison de ceux-ci pour une utilisation dans le traitement thérapeutique d'un chien où la neutralisation d'une cible est souhaitée en l'absence de fonction effectrice non souhaitable, ledit anticorps, ladite protéine de fusion ou ledit fragment de liaison ayant un domaine constant de chaîne lourde comprenant la séquence d'acides aminés de SEQ ID N° : 8, SEQ ID N° : 11 ou SEQ ID N° : 13, où la séquence d'acides aminés de la chaîne lourde minimise l'activation de fonctions effectrices du système immunitaire en aval lorsque l'anticorps, la protéine de fusion ou le fragment de liaison est lié(e) à son antigène cible.

2. L'anticorps, la protéine de fusion ou le fragment de liaison pour une utilisation telle que revendiquée dans la revendication 1 où le traitement thérapeutique du chien se rapporte au traitement, à l'inhibition ou à l'amélioration d'une douleur ou d'une inflammation chez le chien.

3. L'anticorps, la protéine de fusion ou le fragment de liaison pour une utilisation telle que revendiquée dans la revendication 1 ou la revendication 2 où le traitement thérapeutique du chien se rapporte au traitement de l'arthrite ou d'une affection arthritique.

4. L'anticorps, la protéine de fusion ou le fragment de liaison pour une utilisation telle que revendiquée dans la revendication 2 où la douleur est sélectionnée dans le groupe consistant en une douleur neuropathique, une douleur oncologique, une douleur associée à, ou résultant de, l'arthrite rhumatoïde, une douleur associée à, ou résultant de, l'ostéoarthrite, une douleur associée à, ou résultant de, une inflammation et une douleur associée au, ou résultant du prurit.

5. L'anticorps, la protéine de fusion ou le fragment de liaison pour une utilisation telle que revendiquée dans une quelconque des revendications 1 à 4 où les fonctions effectrices du système immunitaire en aval sont sélectionnées dans le groupe consistant en la cytotoxicité dépendante du complément, la cytotoxicité à médiation cellulaire dépendante des anticorps et la pathogenèse cellulaire dépendante des anticorps.

6. L'anticorps, la protéine de fusion ou le fragment de liaison pour une utilisation telle que revendiquée dans la revendication 5 où l'anticorps a une chaîne lourde comprenant la séquence d'acides aminés de SEQ ID N° : 6.

7. L'anticorps, la protéine de fusion ou le fragment de liaison pour une utilisation telle que revendiquée dans une quelconque des revendications 1 à 6 où l'antigène cible est sélectionné dans le groupe consistant en une cytokine, une chimiokine, un facteur de croissance, un récepteur de surface cellulaire, un virus et un composant de la cascade du complément.

8. Un anticorps, une protéine de fusion ou un fragment de liaison de ceux-ci pour une utilisation dans le traitement thérapeutique d'un chien où la destruction d'une cible est souhaitée, ledit anticorps, ladite protéine de fusion ou ledit fragment de liaison ayant un domaine constant de chaîne lourde comprenant la séquence d'acides aminés de SEQ ID N° : 9, SEQ ID N° : 10, ou SEQ ID N° : 14 où la séquence d'acides aminés de la chaîne lourde médie l'activation de fonctions effectrices du système immunitaire en aval lorsque l'anticorps, la protéine de fusion ou le fragment de liaison est lié(e) à son antigène cible.

9. L'anticorps, la protéine de fusion ou le fragment de liaison pour une utilisation telle que revendiquée dans la revendication 8 où le traitement thérapeutique se rapporte au traitement d'un cancer ou d'une maladie infectieuse chez le chien.

10. L'anticorps, la protéine de fusion ou le fragment de liaison pour une utilisation telle que revendiquée dans la revendication 9 où les fonctions effectrices du système immunitaire en aval sont sélectionnées dans le groupe consistant en la cytotoxicité dépendante du complément, la cytotoxicité à médiation cellulaire dépendante des anticorps et la pathogenèse cellulaire dépendante des anticorps.

11. L'anticorps, la protéine de fusion ou le fragment de liaison pour une utilisation telle que revendiquée dans la revendication 9 ou la revendication 10 où l'antigène cible est un antigène spécifique d'un cancer.

12. L'anticorps, la protéine de fusion ou le fragment de liaison pour une utilisation telle que revendiquée dans la revendication 11 où l'antigène spécifique d'un cancer est sélectionné dans le groupe consistant en une cytokine, une chimiokine, un facteur de croissance, un récepteur de surface cellulaire, un virus et un composant de la cascade du complément.

13. L'anticorps, la protéine de fusion ou le fragment de liaison pour utilisation telle que revendiquée dans la revendication 11 où l'antigène spécifique d'un cancer est sélectionné dans le groupe consistant en protéines CD2, CD4, CD8, CD20, EGFR, VEGFR et HER2.


### SEQUENCE LISTING

<110> NVIP Pty Ltd
<120> Therapeutic Canine Immunoglobulins and Methods of Using the Same
<130> P122572.WO.01
<150> GB1114858.2
   <151> 2011-08-29
<150> US61/483481
   <151> 2011-05-06
<150> US61/531439
   <151> 2011-09-06
<160> 15
<170> PatentIn version 3.5
<210> 1
   <211> 453
   <212> PRT
   <213> Artificial Sequence
<220>
   <223> Caninised anti-NGF VH canine IgG-A heavy chain (caN-HCA)
<400> 1
   Glu Val Gln Leu Val Glu Ser Gly Gly Asp Leu Val Asn Pro Gly Gly
   1               5                   10                  15
   Thr Leu Thr Leu Ser Cys Val Val Ser Gly Phe Ser Leu Thr Asn Asn
               20                  25                  30
   Asn Val Asn Trp Val Arg Gln Ala Leu Gly Arg Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Leu Thr Ile Thr Arg Asp Thr Ser Lys Ser Thr Val Phe Leu
   65                  70                  75                  80
   Lys Met His Ser Leu Gln Ser Glu Asp Thr Ala Thr Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Leu Val Thr Val Ser Ser Ala Ser Thr Thr Ala Pro
           115                 120                 125
   Ser Val Phe Pro Leu Ala Pro Ser Cys Gly Ser Thr Ser Gly Ser Thr
       130                 135                 140
   Val Ala Leu Ala Cys Leu Val Ser Gly Tyr Phe Pro Glu Pro Val Thr
   145                 150                 155                 160
   Val Ser Trp Asn Ser Gly Ser Leu Thr Ser Gly Val His Thr Phe Pro
                   165                 170                 175
   Ser Val Leu Gln Ser Ser Gly Leu His Ser Leu Ser Ser Met Val Thr
               180                 185                 190
   Val Pro Ser Ser Arg Trp Pro Ser Glu Thr Phe Thr Cys Asn Val Val
           195                 200                 205
   His Pro Ala Ser Asn Thr Lys Val Asp Lys Pro Val Phe Asn Glu Cys
       210                 215                 220
   Arg Cys Thr Asp Thr Pro Pro Cys Pro Val Pro Glu Pro Leu Gly Gly
   225                 230                 235                 240
   Pro Ser Val Leu Ile Phe Pro Pro Lys Pro Lys Asp Ile Leu Arg Ile
                   245                 250                 255
   Thr Arg Thr Pro Glu Val Thr Cys Val Val Leu Asp Leu Gly Arg Glu
               260                 265                 270
   Asp Pro Glu Val Gln Ile Ser Trp Phe Val Asp Gly Lys Glu Val His
           275                 280                 285
   Thr Ala Lys Thr Gln Ser Arg Glu Gln Gln Phe Asn Gly Thr Tyr Arg
       290                 295                 300
   Val Val Ser Val Leu Pro Ile Glu His Gln Asp Trp Leu Thr Gly Lys
   305                 310                 315                 320
   Glu Phe Lys Cys Arg Val Asn His Ile Asp Leu Pro Ser Pro Ile Glu
                   325                 330                 335
   Arg Thr Ile Ser Lys Ala Arg Gly Arg Ala His Lys Pro Ser Val Tyr
               340                 345                 350
   Val Leu Pro Pro Ser Pro Lys Glu Leu Ser Ser Ser Asp Thr Val Ser
           355                 360                 365
   Ile Thr Cys Leu Ile Lys Asp Phe Tyr Pro Pro Asp Ile Asp Val Glu
       370                 375                 380
   Trp Gln Ser Asn Gly Gln Gln Glu Pro Glu Arg Lys His Arg Met Thr
   385 390 395 400
   Pro Pro Gln Leu Asp Glu Asp Gly Ser Tyr Phe Leu Tyr Ser Lys Leu
   405 410 415
   Ser Val Asp Lys Ser Arg Trp Gln Gln Gly Asp Pro Phe Thr Cys Ala
   420 425 430
   Val Met His Glu Thr Leu Gln Asn His Tyr Thr Asp Leu Ser Leu Ser
   435 440 445
   His Ser Pro Gly Lys
   450
<210> 2
   <211> 457
   <212> PRT
   <213> Artificial Sequence
<220>
   <223> Caninised anti-NGF VH canine IgG-B heavy chain (caN-HCB)
<400> 2
   Glu Val Gln Leu Val Glu Ser Gly Gly Asp Leu Val Asn Pro Gly Gly
   1 5 10 15
   Thr Leu Thr Leu Ser Cys Val Val Ser Gly Phe Ser Leu Thr Asn Asn
   20 25 30
   Asn Val Asn Trp Val Arg Gln Ala Leu Gly Arg Gly Leu Glu Trp Val
   35 40 45
   Gly Gly Val Trp Ala Gly Gly Ala Thr Asp Tyr Asn Ser Ala Leu Lys
   50 55 60
   Ser Arg Leu Thr Ile Thr Arg Asp Thr Ser Lys Ser Thr Val Phe Leu
   65 70 75 80
   Lys Met His Ser Leu Gln Ser Glu Asp Thr Ala Thr Tyr Tyr Cys Ala
   85 90 95
   Arg Asp Gly Gly Tyr Ser Ser Ser Thr Leu Tyr Ala Met Asp Ala Trp
   100 105 110
   Gly Gln Gly Thr Leu Val Thr Val Ser Ser Ala Ser Thr Thr Ala Pro
   115 120 125
   Ser Val Phe Pro Leu Ala Pro Ser Cys Gly Ser Thr Ser Gly Ser Thr
       130                 135                 140
   Val Ala Leu Ala Cys Leu Val Ser Gly Tyr Phe Pro Glu Pro Val Thr
   145                 150                 155                 160
   Val Ser Trp Asn Ser Gly Ser Leu Thr Ser Gly Val His Thr Phe Pro
                   165                 170                 175
   Ser Val Leu Gln Ser Ser Gly Leu Tyr Ser Leu Ser Ser Met Val Thr
               180                 185                 190
   Val Pro Ser Ser Arg Trp Pro Ser Glu Thr Phe Thr Cys Asn Val Ala
           195                 200                 205
   His Pro Ala Ser Lys Thr Lys Val Asp Lys Pro Val Pro Lys Arg Glu
       210                 215                 220
   Asn Gly Arg Val Pro Arg Pro Pro Asp Cys Pro Lys Cys Pro Ala Pro
   225                 230                 235                 240
   Glu Met Leu Gly Gly Pro Ser Val Phe Ile Phe Pro Pro Lys Pro Lys
                   245                 250                 255
   Asp Thr Leu Leu Ile Ala Arg Thr Pro Glu Val Thr Cys Val Val Val
               260                 265                 270
   Asp Leu Asp Pro Glu Asp Pro Glu Val Gln Ile Ser Trp Phe Val Asp
           275                 280                 285
   Gly Lys Gln Met Gln Thr Ala Lys Thr Gln Pro Arg Glu Glu Gln Phe
       290                 295                 300
   Asn Gly Thr Tyr Arg Val Val Ser Val Leu Pro Ile Gly His Gln Asp
   305                 310                 315                 320
   Trp Leu Lys Gly Lys Gln Phe Thr Cys Lys Val Asn Asn Lys Ala Leu
                   325                 330                 335
   Pro Ser Pro Ile Glu Arg Thr Ile Ser Lys Ala Arg Gly Gln Ala His
               340                 345                 350
   Gln Pro Ser Val Tyr Val Leu Pro Pro Ser Arg Glu Glu Leu Ser Lys
           355                 360                 365
   Asn Thr Val Ser Leu Thr Cys Leu Ile Lys Asp Phe Tyr Pro Pro Asp
       370                 375                 380
   Ile Asp Val Glu Trp Gln Ser Asn Gly Gln Gln Glu Pro Glu Ser Lys
   385                 390                 395                 400
   Tyr Arg Thr Thr Pro Pro Gln Leu Asp Glu Asp Gly Ser Tyr Phe Leu
                   405                 410                 415
   Tyr Ser Lys Leu Ser Val Asp Lys Ser Arg Trp Gln Arg Gly Asp Thr
               420                 425                 430
   Phe Ile Cys Ala Val Met His Glu Ala Leu His Asn His Tyr Thr Gln
           435                 440                 445
   Glu Ser Leu Ser His Ser Pro Gly Lys
       450                 455
<210> 3
   <211> 455
   <212> PRT
   <213> Artificial Sequence
<220>
   <223> Caninised anti-NGF VH canine IgG-C heavy chain (caN-HCC)
<400> 3
   Glu Val Gln Leu Val Glu Ser Gly Gly Asp Leu Val Asn Pro Gly Gly
   1               5                   10                  15
   Thr Leu Thr Leu Ser Cys Val Val Ser Gly Phe Ser Leu Thr Asn Asn
               20                  25                  30
   Asn Val Asn Trp Val Arg Gln Ala Leu Gly Arg Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Leu Thr Ile Thr Arg Asp Thr Ser Lys Ser Thr Val Phe Leu
   65                  70                  75                  80
   Lys Met His Ser Leu Gln Ser Glu Asp Thr Ala Thr Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Leu Val Thr Val Ser Ser Ala Ser Thr Thr Ala Pro
           115                 120                 125
   Ser Val Phe Pro Leu Ala Pro Ser Cys Gly Ser Gln Ser Gly Ser Thr
       130                 135                 140
   Val Ala Leu Ala Cys Leu Val Ser Gly Tyr Ile Pro Glu Pro Val Thr
   145                 150                 155                 160
   Val Ser Trp Asn Ser Val Ser Leu Thr Ser Gly Val His Thr Phe Pro
                   165                 170                 175
   Ser Val Leu Gln Ser Ser Gly Leu Tyr Ser Leu Ser Ser Met Val Thr
               180                 185                 190
   Val Pro Ser Ser Arg Trp Pro Ser Glu Thr Phe Thr Cys Asn Val Ala
           195                 200                 205
   His Pro Ala Thr Asn Thr Lys Val Asp Lys Pro Val Ala Lys Glu Cys
       210                 215                 220
   Glu Cys Lys Cys Asn Cys Asn Asn Cys Pro Cys Pro Gly Cys Gly Leu
   225                 230                 235                 240
   Leu Gly Gly Pro Ser Val Phe Ile Phe Pro Pro Lys Pro Lys Asp Ile
                   245                 250                 255
   Leu Val Thr Ala Arg Thr Pro Thr Val Thr Cys Val Val Val Asp Leu
               260                 265                 270
   Asp Pro Glu Asn Pro Glu Val Gln Ile Ser Trp Phe Val Asp Ser Lys
           275                 280                 285
   Gln Val Gln Thr Ala Asn Thr Gln Pro Arg Glu Glu Gln Ser Asn Gly
       290                 295                 300
   Thr Tyr Arg Val Val Ser Val Leu Pro Ile Gly His Gln Asp Trp Leu
   305                 310                 315                 320
   Ser Gly Lys Gln Phe Lys Cys Lys Val Asn Asn Lys Ala Leu Pro Ser
                   325                 330                 335
   Pro Ile Glu Glu Ile Ile Ser Lys Thr Pro Gly Gln Ala His Gln Pro
               340                 345                 350
   Asn Val Tyr Val Leu Pro Pro Ser Arg Asp Glu Met Ser Lys Asn Thr
           355                 360                 365
   Val Thr Leu Thr Cys Leu Val Lys Asp Phe Phe Pro Pro Glu Ile Asp
       370                 375                 380
   Val Glu Trp Gln Ser Asn Gly Gln Gln Glu Pro Glu Ser Lys Tyr Arg
   385                 390                 395                 400
   Met Thr Pro Pro Gln Leu Asp Glu Asp Gly Ser Tyr Phe Leu Tyr Ser
                   405                 410                 415
   Lys Leu Ser Val Asp Lys Ser Arg Trp Gln Arg Gly Asp Thr Phe Ile
               420                 425                 430
   Cys Ala Val Met His Glu Ala Leu His Asn His Tyr Thr Gln Ile Ser
           435                 440                 445
   Leu Ser His Ser Pro Gly Lys
       450                 455
<210> 4
   <211> 453
   <212> PRT
   <213> Artificial Sequence
<220>
   <223> Caninised anti-NGF VH canine IgG-D heavy chain (caN-HCD)
<400> 4
   Glu Val Gln Leu Val Glu Ser Gly Gly Asp Leu Val Asn Pro Gly Gly
   1               5                   10                  15
   Thr Leu Thr Leu Ser Cys Val Val Ser Gly Phe Ser Leu Thr Asn Asn
               20                  25                  30
   Asn Val Asn Trp Val Arg Gln Ala Leu Gly Arg Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Leu Thr Ile Thr Arg Asp Thr Ser Lys Ser Thr Val Phe Leu
   65                  70                  75                  80
   Lys Met His Ser Leu Gln Ser Glu Asp Thr Ala Thr Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Leu Val Thr Val Ser Ser Ala Ser Thr Thr Ala Pro
           115                 120                 125
   Ser Val Phe Pro Leu Ala Pro Ser Cys Gly Ser Thr Ser Gly Ser Thr
       130                 135                 140
   Val Ala Leu Ala Cys Leu Val Ser Gly Tyr Phe Pro Glu Pro Val Thr
   145                 150                 155                 160
   Val Ser Trp Asn Ser Gly Ser Leu Thr Ser Gly Val His Thr Phe Pro
                   165                 170                 175
   Ser Val Leu Gln Ser Ser Gly Leu Tyr Ser Leu Ser Ser Thr Val Thr
               180                 185                 190
   Val Pro Ser Ser Arg Trp Pro Ser Glu Thr Phe Thr Cys Asn Val Val
           195                 200                 205
   His Pro Ala Ser Asn Thr Lys Val Asp Lys Pro Val Pro Lys Glu Ser
       210                 215                 220
   Thr Cys Lys Cys Ile Ser Pro Cys Pro Val Pro Glu Ser Leu Gly Gly
   225                 230                 235                 240
   Pro Ser Val Phe Ile Phe Pro Pro Lys Pro Lys Asp Ile Leu Arg Ile
                   245                 250                 255
   Thr Arg Thr Pro Glu Ile Thr Cys Val Val Leu Asp Leu Gly Arg Glu
               260                 265                 270
   Asp Pro Glu Val Gln Ile Ser Trp Phe Val Asp Gly Lys Glu Val His
           275                 280                 285
   Thr Ala Lys Thr Gln Pro Arg Glu Gln Gln Phe Asn Ser Thr Tyr Arg
       290                 295                 300
   Val Val Ser Val Leu Pro Ile Glu His Gln Asp Trp Leu Thr Gly Lys
   305                 310                 315                 320
   Glu Phe Lys Cys Arg Val Asn His Ile Gly Leu Pro Ser Pro Ile Glu
                   325                 330                 335
   Arg Thr Ile Ser Lys Ala Arg Gly Gln Ala His Gln Pro Ser Val Tyr
               340                 345                 350
   Val Leu Pro Pro Ser Pro Lys Glu Leu Ser Ser Ser Asp Thr Val Thr
           355                 360                 365
   Leu Thr Cys Leu Ile Lys Asp Phe Tyr Pro Pro Glu Ile Asp Val Glu
       370                 375                 380
   Trp Gln Ser Asn Gly Gln Pro Glu Pro Glu Ser Lys Tyr His Thr Thr
   385 390 395 400
   Ala Pro Gln Leu Asp Glu Asp Gly Ser Tyr Phe Leu Tyr Ser Lys Leu
   405 410 415
   Ser Val Asp Lys Ser Arg Trp Gln Gln Gly Asp Thr Phe Thr Cys Ala
   420 425 430
   Val Met His Glu Ala Leu Gln Asn His Tyr Thr Asp Leu Ser Leu Ser
   435 440 445
   His Ser Pro Gly Lys
   450
<210> 5
   <211> 217
   <212> PRT
   <213> Artificial Sequence
<220>
   <223> Caninised anti-NGF VL canine kappa light chain (caN-kLC)
<400> 5
   Asp Ile Gln Met Thr Gln Ser Pro Ala Ser Leu Ser Leu Ser Gln Glu
   1 5 10 15
   Glu Lys Val Thr Ile Thr Cys Arg Ala Ser Glu Asp Ile Tyr Asn Ala
   20 25 30
   Leu Ala Trp Tyr Gln Gln Lys Pro Gly Gln Ala Pro Lys Leu Leu Ile
   35 40 45
   Tyr Asn Thr Asp Thr Leu His Thr Gly Val Pro Ser Arg Phe Ser Gly
   50 55 60
   Ser Gly Ser Gly Thr Glu Tyr Ser Leu Thr Ile Asn Ser Leu Glu Ser
   65 70 75 80
   Glu Asp Val Ala Val Tyr Phe Cys Gln His Tyr Phe His Tyr Pro Arg
   85 90 95
   Thr Phe Gly Ala Gly Thr Lys Val Glu Leu Lys Arg Asn Asp Ala Gln
   100 105 110
   Pro Ala Val Tyr Leu Phe Gln Pro Ser Pro Asp Gln Leu His Thr Gly
   115 120 125
   Ser Ala Ser Val Val Cys Leu Leu Asn Ser Phe Tyr Pro Lys Asp Ile
       130                 135                 140
   Asn Val Lys Trp Lys Val Asp Gly Val Ile Gln Asp Thr Gly Ile Gln
   145                 150                 155                 160
   Glu Ser Val Thr Glu Gln Asp Lys Asp Ser Thr Tyr Ser Leu Ser Ser
                   165                 170                 175
   Thr Leu Thr Met Ser Ser Thr Glu Tyr Leu Ser His Glu Leu Tyr Ser
               180                 185                 190
   Cys Glu Ile Thr His Lys Ser Leu Pro Ser Thr Leu Ile Lys Ser Phe
           195                 200                 205
   Gln Arg Ser Glu Cys Gln Arg Val Asp
       210                 215
<210> 6
   <211> 457
   <212> PRT
   <213> Artificial Sequence
<220>
   <223> Caninised anti-NGF VH Aglycosyl canine immunoglobulin heavy chain type B (caN-HCB*)
<400> 6
   Glu Val Gln Leu Val Glu Ser Gly Gly Asp Leu Val Asn Pro Gly Gly
   1               5                   10                  15
   Thr Leu Thr Leu Ser Cys Val Val Ser Gly Phe Ser Leu Thr Asn Asn
               20                  25                  30
   Asn Val Asn Trp Val Arg Gln Ala Leu Gly Arg Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Leu Thr Ile Thr Arg Asp Thr Ser Lys Ser Thr Val Phe Leu
   65                  70                  75                  80
   Lys Met His Ser Leu Gln Ser Glu Asp Thr Ala Thr Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Leu Val Thr Val Ser Ser Ala Ser Thr Thr Ala Pro
           115                 120                 125
   Ser Val Phe Pro Leu Ala Pro Ser Cys Gly Ser Thr Ser Gly Ser Thr
       130                 135                 140
   Val Ala Leu Ala Cys Leu Val Ser Gly Tyr Phe Pro Glu Pro Val Thr
   145                 150                 155                 160
   Val Ser Trp Asn Ser Gly Ser Leu Thr Ser Gly Val His Thr Phe Pro
                   165                 170                 175
   Ser Val Leu Gln Ser Ser Gly Leu Tyr Ser Leu Ser Ser Met Val Thr
               180                 185                 190
   Val Pro Ser Ser Arg Trp Pro Ser Glu Thr Phe Thr Cys Asn Val Ala
           195                 200                 205
   His Pro Ala Ser Lys Thr Lys Val Asp Lys Pro Val Pro Lys Arg Glu
       210                 215                 220
   Asn Gly Arg Val Pro Arg Pro Pro Asp Cys Pro Lys Cys Pro Ala Pro
   225                 230                 235                 240
   Glu Met Leu Gly Gly Pro Ser Val Phe Ile Phe Pro Pro Lys Pro Lys
                   245                 250                 255
   Asp Thr Leu Leu Ile Ala Arg Thr Pro Glu Val Thr Cys Val Val Val
               260                 265                 270
   Asp Leu Asp Pro Glu Asp Pro Glu Val Gln Ile Ser Trp Phe Val Asp
           275                 280                 285
   Gly Lys Gln Met Gln Thr Ala Lys Thr Gln Pro Arg Glu Glu Gln Phe
       290                 295                 300
   Ala Gly Thr Tyr Arg Val Val Ser Val Leu Pro Ile Gly His Gln Asp
   305                 310                 315                 320
   Trp Leu Lys Gly Lys Gln Phe Thr Cys Lys Val Asn Asn Lys Ala Leu
                   325                 330                 335
   Pro Ser Pro Ile Glu Arg Thr Ile Ser Lys Ala Arg Gly Gln Ala His
               340                 345                 350
   Gln Pro Ser Val Tyr Val Leu Pro Pro Ser Arg Glu Glu Leu Ser Lys
           355                 360                 365
   Asn Thr Val Ser Leu Thr Cys Leu Ile Lys Asp Phe Tyr Pro Pro Asp
       370                 375                 380
   Ile Asp Val Glu Trp Gln Ser Asn Gly Gln Gln Glu Pro Glu Ser Lys
   385                 390                 395                 400
   Tyr Arg Thr Thr Pro Pro Gln Leu Asp Glu Asp Gly Ser Tyr Phe Leu
                   405                 410                 415
   Tyr Ser Lys Leu Ser Val Asp Lys Ser Arg Trp Gln Arg Gly Asp Thr
               420                 425                 430
   Phe Ile Cys Ala Val Met His Glu Ala Leu His Asn His Tyr Thr Gln
           435                 440                 445
   Glu Ser Leu Ser His Ser Pro Gly Lys
       450                 455
<210> 7
   <211> 455
   <212> PRT
   <213> Artificial Sequence
<220>
   <223> Caninised anti-NGF VH aglycosyl canine immunoglobulin heavy chain type C (caN-HCC*)
<400> 7
   Glu Val Gln Leu Val Glu Ser Gly Gly Asp Leu Val Asn Pro Gly Gly
   1               5                   10                  15
   Thr Leu Thr Leu Ser Cys Val Val Ser Gly Phe Ser Leu Thr Asn Asn
               20                  25                  30
   Asn Val Asn Trp Val Arg Gln Ala Leu Gly Arg Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Leu Thr Ile Thr Arg Asp Thr Ser Lys Ser Thr Val Phe Leu
   65                  70                  75                  80
   Lys Met His Ser Leu Gln Ser Glu Asp Thr Ala Thr Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Leu Val Thr Val Ser Ser Ala Ser Thr Thr Ala Pro
           115                 120                 125
   Ser Val Phe Pro Leu Ala Pro Ser Cys Gly Ser Gln Ser Gly Ser Thr
       130                 135                 140
   Val Ala Leu Ala Cys Leu Val Ser Gly Tyr Ile Pro Glu Pro Val Thr
   145                 150                 155                 160
   Val Ser Trp Asn Ser Val Ser Leu Thr Ser Gly Val His Thr Phe Pro
                   165                 170                 175
   Ser Val Leu Gln Ser Ser Gly Leu Tyr Ser Leu Ser Ser Met Val Thr
               180                 185                 190
   Val Pro Ser Ser Arg Trp Pro Ser Glu Thr Phe Thr Cys Asn Val Ala
           195                 200                 205
   His Pro Ala Thr Asn Thr Lys Val Asp Lys Pro Val Ala Lys Glu Cys
       210                 215                 220
   Glu Cys Lys Cys Asn Cys Asn Asn Cys Pro Cys Pro Gly Cys Gly Leu
   225                 230                 235                 240
   Leu Gly Gly Pro Ser Val Phe Ile Phe Pro Pro Lys Pro Lys Asp Ile
                   245                 250                 255
   Leu Val Thr Ala Arg Thr Pro Thr Val Thr Cys Val Val Val Asp Leu
               260                 265                 270
   Asp Pro Glu Asn Pro Glu Val Gln Ile Ser Trp Phe Val Asp Ser Lys
           275                 280                 285
   Gln Val Gln Thr Ala Asn Thr Gln Pro Arg Glu Glu Gln Ser Ala Gly
       290                 295                 300
   Thr Tyr Arg Val Val Ser Val Leu Pro Ile Gly His Gln Asp Trp Leu
   305                 310                 315                 320
   Ser Gly Lys Gln Phe Lys Cys Lys Val Asn Asn Lys Ala Leu Pro Ser
                   325                 330                 335
   Pro Ile Glu Glu Ile Ile Ser Lys Thr Pro Gly Gln Ala His Gln Pro
               340                 345                 350
   Asn Val Tyr Val Leu Pro Pro Ser Arg Asp Glu Met Ser Lys Asn Thr
           355                 360                 365
   Val Thr Leu Thr Cys Leu Val Lys Asp Phe Phe Pro Pro Glu Ile Asp
   370 375 380
   Val Glu Trp Gln Ser Asn Gly Gln Gln Glu Pro Glu Ser Lys Tyr Arg
   385 390 395 400
   Met Thr Pro Pro Gln Leu Asp Glu Asp Gly Ser Tyr Phe Leu Tyr Ser
   405 410 415
   Lys Leu Ser Val Asp Lys Ser Arg Trp Gln Arg Gly Asp Thr Phe Ile
   420 425 430
   Cys Ala Val Met His Glu Ala Leu His Asn His Tyr Thr Gln Ile Ser
   435 440 445
   Leu Ser His Ser Pro Gly Lys
   450 455
<210> 8
   <211> 331
   <212> PRT
   <213> Artificial Sequence
<220>
   <223> Canine immunoglobulin heavy chain type A Fc domain (HCA)
<400> 8
   Ala Ser Thr Thr Ala Pro Ser Val Phe Pro Leu Ala Pro Ser Cys Gly
   1 5 10 15
   Ser Thr Ser Gly Ser Thr Val Ala Leu Ala Cys Leu Val Ser Gly Tyr
   20 25 30
   Phe Pro Glu Pro Val Thr Val Ser Trp Asn Ser Gly Ser Leu Thr Ser
   35 40 45
   Gly Val His Thr Phe Pro Ser Val Leu Gln Ser Ser Gly Leu His Ser
   50 55 60
   Leu Ser Ser Met Val Thr Val Pro Ser Ser Arg Trp Pro Ser Glu Thr
   65 70 75 80
   Phe Thr Cys Asn Val Val His Pro Ala Ser Asn Thr Lys Val Asp Lys
   85 90 95
   Pro Val Phe Asn Glu Cys Arg Cys Thr Asp Thr Pro Pro Cys Pro Val
   100 105 110
   Pro Glu Pro Leu Gly Gly Pro Ser Val Leu Ile Phe Pro Pro Lys Pro
           115                 120                 125
   Lys Asp Ile Leu Arg Ile Thr Arg Thr Pro Glu Val Thr Cys Val Val
       130                 135                 140
   Leu Asp Leu Gly Arg Glu Asp Pro Glu Val Gln Ile Ser Trp Phe Val
   145                 150                 155                 160
   Asp Gly Lys Glu Val His Thr Ala Lys Thr Gln Ser Arg Glu Gln Gln
                   165                 170                 175
   Phe Asn Gly Thr Tyr Arg Val Val Ser Val Leu Pro Ile Glu His Gln
               180                 185                 190
   Asp Trp Leu Thr Gly Lys Glu Phe Lys Cys Arg Val Asn His Ile Asp
           195                 200                 205
   Leu Pro Ser Pro Ile Glu Arg Thr Ile Ser Lys Ala Arg Gly Arg Ala
       210                 215                 220
   His Lys Pro Ser Val Tyr Val Leu Pro Pro Ser Pro Lys Glu Leu Ser
   225                 230                 235                 240
   Ser Ser Asp Thr Val Ser Ile Thr Cys Leu Ile Lys Asp Phe Tyr Pro
                   245                 250                 255
   Pro Asp Ile Asp Val Glu Trp Gln Ser Asn Gly Gln Gln Glu Pro Glu
               260                 265                 270
   Arg Lys His Arg Met Thr Pro Pro Gln Leu Asp Glu Asp Gly Ser Tyr
           275                 280                 285
   Phe Leu Tyr Ser Lys Leu Ser Val Asp Lys Ser Arg Trp Gln Gln Gly
       290                 295                 300
   Asp Pro Phe Thr Cys Ala Val Met His Glu Thr Leu Gln Asn His Tyr
   305                 310                 315                 320
   Thr Asp Leu Ser Leu Ser His Ser Pro Gly Lys
                   325                 330
<210> 9
   <211> 335
   <212> PRT
   <213> Artificial Sequence
<220>
   <223> Canine immunoglobulin heavy chain type B Fc domain (HCB)
<400> 9
   Ala Ser Thr Thr Ala Pro Ser Val Phe Pro Leu Ala Pro Ser Cys Gly
   1               5                   10                  15
   Ser Thr Ser Gly Ser Thr Val Ala Leu Ala Cys Leu Val Ser Gly Tyr
               20                  25                  30
   Phe Pro Glu Pro Val Thr Val Ser Trp Asn Ser Gly Ser Leu Thr Ser
           35                  40                  45
   Gly Val His Thr Phe Pro Ser Val Leu Gln Ser Ser Gly Leu Tyr Ser
       50                  55                  60
   Leu Ser Ser Met Val Thr Val Pro Ser Ser Arg Trp Pro Ser Glu Thr
   65                  70                  75                  80
   Phe Thr Cys Asn Val Ala His Pro Ala Ser Lys Thr Lys Val Asp Lys
                   85                  90                  95
   Pro Val Pro Lys Arg Glu Asn Gly Arg Val Pro Arg Pro Pro Asp Cys
               100                 105                 110
   Pro Lys Cys Pro Ala Pro Glu Met Leu Gly Gly Pro Ser Val Phe Ile
           115                 120                 125
   Phe Pro Pro Lys Pro Lys Asp Thr Leu Leu Ile Ala Arg Thr Pro Glu
       130                 135                 140
   Val Thr Cys Val Val Val Asp Leu Asp Pro Glu Asp Pro Glu Val Gln
   145                 150                 155                 160
   Ile Ser Trp Phe Val Asp Gly Lys Gln Met Gln Thr Ala Lys Thr Gln
                   165                 170                 175
   Pro Arg Glu Glu Gln Phe Asn Gly Thr Tyr Arg Val Val Ser Val Leu
               180                 185                 190
   Pro Ile Gly His Gln Asp Trp Leu Lys Gly Lys Gln Phe Thr Cys Lys
           195                 200                 205
   Val Asn Asn Lys Ala Leu Pro Ser Pro Ile Glu Arg Thr Ile Ser Lys
       210                 215                 220
   Ala Arg Gly Gln Ala His Gln Pro Ser Val Tyr Val Leu Pro Pro Ser
   225                 230                 235                 240
   Arg Glu Glu Leu Ser Lys Asn Thr Val Ser Leu Thr Cys Leu Ile Lys
   245 250 255
   Asp Phe Tyr Pro Pro Asp Ile Asp Val Glu Trp Gln Ser Asn Gly Gln
   260 265 270
   Gln Glu Pro Glu Ser Lys Tyr Arg Thr Thr Pro Pro Gln Leu Asp Glu
   275 280 285
   Asp Gly Ser Tyr Phe Leu Tyr Ser Lys Leu Ser Val Asp Lys Ser Arg
   290 295 300
   Trp Gln Arg Gly Asp Thr Phe Ile Cys Ala Val Met His Glu Ala Leu
   305 310 315 320
   His Asn His Tyr Thr Gln Glu Ser Leu Ser His Ser Pro Gly Lys
   325 330 335
<210> 10
   <211> 333
   <212> PRT
   <213> Artificial Sequence
<220>
   <223> Canine immunoglobulin heavy chain type C Fc domain (HCC)
<400> 10
   Ala Ser Thr Thr Ala Pro Ser Val Phe Pro Leu Ala Pro Ser Cys Gly
   1 5 10 15
   Ser Gln Ser Gly Ser Thr Val Ala Leu Ala Cys Leu Val Ser Gly Tyr
   20 25 30
   Ile Pro Glu Pro Val Thr Val Ser Trp Asn Ser Val Ser Leu Thr Ser
   35 40 45
   Gly Val His Thr Phe Pro Ser Val Leu Gln Ser Ser Gly Leu Tyr Ser
   50 55 60
   Leu Ser Ser Met Val Thr Val Pro Ser Ser Arg Trp Pro Ser Glu Thr
   65 70 75 80
   Phe Thr Cys Asn Val Ala His Pro Ala Thr Asn Thr Lys Val Asp Lys
   85 90 95
   Pro Val Ala Lys Glu Cys Glu Cys Lys Cys Asn Cys Asn Asn Cys Pro
   100 105 110
   Cys Pro Gly Cys Gly Leu Leu Gly Gly Pro Ser Val Phe Ile Phe Pro
           115                 120                 125
   Pro Lys Pro Lys Asp Ile Leu Val Thr Ala Arg Thr Pro Thr Val Thr
       130                 135                 140
   Cys Val Val Val Asp Leu Asp Pro Glu Asn Pro Glu Val Gln Ile Ser
   145                 150                 155                 160
   Trp Phe Val Asp Ser Lys Gln Val Gln Thr Ala Asn Thr Gln Pro Arg
                   165                 170                 175
   Glu Glu Gln Ser Asn Gly Thr Tyr Arg Val Val Ser Val Leu Pro Ile
               180                 185                 190
   Gly His Gln Asp Trp Leu Ser Gly Lys Gln Phe Lys Cys Lys Val Asn
           195                 200                 205
   Asn Lys Ala Leu Pro Ser Pro Ile Glu Glu Ile Ile Ser Lys Thr Pro
       210                 215                 220
   Gly Gln Ala His Gln Pro Asn Val Tyr Val Leu Pro Pro Ser Arg Asp
   225                 230                 235                 240
   Glu Met Ser Lys Asn Thr Val Thr Leu Thr Cys Leu Val Lys Asp Phe
                   245                 250                 255
   Phe Pro Pro Glu Ile Asp Val Glu Trp Gln Ser Asn Gly Gln Gln Glu
               260                 265                 270
   Pro Glu Ser Lys Tyr Arg Met Thr Pro Pro Gln Leu Asp Glu Asp Gly
           275                 280                 285
   Ser Tyr Phe Leu Tyr Ser Lys Leu Ser Val Asp Lys Ser Arg Trp Gln
       290                 295                 300
   Arg Gly Asp Thr Phe Ile Cys Ala Val Met His Glu Ala Leu His Asn
   305                 310                 315                 320
   His Tyr Thr Gln Ile Ser Leu Ser His Ser Pro Gly Lys
                   325                 330
<210> 11
   <211> 331
   <212> PRT
   <213> Artificial Sequence
<220>
   <223> Canine immunoglobulin heavy chain type D Fc domain (HCD)
<400> 11
   Ala Ser Thr Thr Ala Pro Ser Val Phe Pro Leu Ala Pro Ser Cys Gly
   1               5                   10                  15
   Ser Thr Ser Gly Ser Thr Val Ala Leu Ala Cys Leu Val Ser Gly Tyr
               20                  25                  30
   Phe Pro Glu Pro Val Thr Val Ser Trp Asn Ser Gly Ser Leu Thr Ser
           35                  40                  45
   Gly Val His Thr Phe Pro Ser Val Leu Gln Ser Ser Gly Leu Tyr Ser
       50                  55                  60
   Leu Ser Ser Thr Val Thr Val Pro Ser Ser Arg Trp Pro Ser Glu Thr
   65                  70                  75                  80
   Phe Thr Cys Asn Val Val His Pro Ala Ser Asn Thr Lys Val Asp Lys
                   85                  90                  95
   Pro Val Pro Lys Glu Ser Thr Cys Lys Cys Ile Ser Pro Cys Pro Val
               100                 105                 110
   Pro Glu Ser Leu Gly Gly Pro Ser Val Phe Ile Phe Pro Pro Lys Pro
           115                 120                 125
   Lys Asp Ile Leu Arg Ile Thr Arg Thr Pro Glu Ile Thr Cys Val Val
       130                 135                 140
   Leu Asp Leu Gly Arg Glu Asp Pro Glu Val Gln Ile Ser Trp Phe Val
   145                 150                 155                 160
   Asp Gly Lys Glu Val His Thr Ala Lys Thr Gln Pro Arg Glu Gln Gln
                   165                 170                 175
   Phe Asn Ser Thr Tyr Arg Val Val Ser Val Leu Pro Ile Glu His Gln
               180                 185                 190
   Asp Trp Leu Thr Gly Lys Glu Phe Lys Cys Arg Val Asn His Ile Gly
           195                 200                 205
   Leu Pro Ser Pro Ile Glu Arg Thr Ile Ser Lys Ala Arg Gly Gln Ala
       210                 215                 220
   His Gln Pro Ser Val Tyr Val Leu Pro Pro Ser Pro Lys Glu Leu Ser
   225                 230                 235                 240
   Ser Ser Asp Thr Val Thr Leu Thr Cys Leu Ile Lys Asp Phe Tyr Pro
                   245                 250                 255
   Pro Glu Ile Asp Val Glu Trp Gln Ser Asn Gly Gln Pro Glu Pro Glu
               260                 265                 270
   Ser Lys Tyr His Thr Thr Ala Pro Gln Leu Asp Glu Asp Gly Ser Tyr
           275                 280                 285
   Phe Leu Tyr Ser Lys Leu Ser Val Asp Lys Ser Arg Trp Gln Gln Gly
       290                 295                 300
   Asp Thr Phe Thr Cys Ala Val Met His Glu Ala Leu Gln Asn His Tyr
   305                 310                 315                 320
   Thr Asp Leu Ser Leu Ser His Ser Pro Gly Lys
                   325                 330
<210> 12
   <211> 331
   <212> PRT
   <213> Artificial Sequence
<220>
   <223> Aglycosyl canine immunoglobulin heavy chain type A Fc domain (HCA*)
<400> 12
   Ala Ser Thr Thr Ala Pro Ser Val Phe Pro Leu Ala Pro Ser Cys Gly
   1               5                   10                  15
   Ser Thr Ser Gly Ser Thr Val Ala Leu Ala Cys Leu Val Ser Gly Tyr
               20                  25                  30
   Phe Pro Glu Pro Val Thr Val Ser Trp Asn Ser Gly Ser Leu Thr Ser
           35                  40                  45
   Gly Val His Thr Phe Pro Ser Val Leu Gln Ser Ser Gly Leu His Ser
       50                  55                  60
   Leu Ser Ser Met Val Thr Val Pro Ser Ser Arg Trp Pro Ser Glu Thr
   65                  70                  75                  80
   Phe Thr Cys Asn Val Val His Pro Ala Ser Asn Thr Lys Val Asp Lys
                   85                  90                  95
   Pro Val Phe Asn Glu Cys Arg Cys Thr Asp Thr Pro Pro Cys Pro Val
               100                 105                 110
   Pro Glu Pro Leu Gly Gly Pro Ser Val Leu Ile Phe Pro Pro Lys Pro
           115                 120                 125
   Lys Asp Ile Leu Arg Ile Thr Arg Thr Pro Glu Val Thr Cys Val Val
       130                 135                 140
   Leu Asp Leu Gly Arg Glu Asp Pro Glu Val Gln Ile Ser Trp Phe Val
   145                 150                 155                 160
   Asp Gly Lys Glu Val His Thr Ala Lys Thr Gln Ser Arg Glu Gln Gln
                   165                 170                 175
   Phe Ala Gly Thr Tyr Arg Val Val Ser Val Leu Pro Ile Glu His Gln
               180                 185                 190
   Asp Trp Leu Thr Gly Lys Glu Phe Lys Cys Arg Val Asn His Ile Asp
           195                 200                 205
   Leu Pro Ser Pro Ile Glu Arg Thr Ile Ser Lys Ala Arg Gly Arg Ala
       210                 215                 220
   His Lys Pro Ser Val Tyr Val Leu Pro Pro Ser Pro Lys Glu Leu Ser
   225                 230                 235                 240
   Ser Ser Asp Thr Val Ser Ile Thr Cys Leu Ile Lys Asp Phe Tyr Pro
                   245                 250                 255
   Pro Asp Ile Asp Val Glu Trp Gln Ser Asn Gly Gln Gln Glu Pro Glu
               260                 265                 270
   Arg Lys His Arg Met Thr Pro Pro Gln Leu Asp Glu Asp Gly Ser Tyr
           275                 280                 285
   Phe Leu Tyr Ser Lys Leu Ser Val Asp Lys Ser Arg Trp Gln Gln Gly
       290                 295                 300
   Asp Pro Phe Thr Cys Ala Val Met His Glu Thr Leu Gln Asn His Tyr
   305                 310                 315                 320
   Thr Asp Leu Ser Leu Ser His Ser Pro Gly Lys
                   325                 330
<210> 13
   <211> 335
   <212> PRT
   <213> Artificial Sequence
<220>
   <223> Aglycosyl caninised immunoglobulin heavy chain type B Fc domain (HCB*)
<400> 13
   Ala Ser Thr Thr Ala Pro Ser Val Phe Pro Leu Ala Pro Ser Cys Gly
   1               5                   10                  15
   Ser Thr Ser Gly Ser Thr Val Ala Leu Ala Cys Leu Val Ser Gly Tyr
               20                  25                  30
   Phe Pro Glu Pro Val Thr Val Ser Trp Asn Ser Gly Ser Leu Thr Ser
           35                  40                  45
   Gly Val His Thr Phe Pro Ser Val Leu Gln Ser Ser Gly Leu Tyr Ser
       50                  55                  60
   Leu Ser Ser Met Val Thr Val Pro Ser Ser Arg Trp Pro Ser Glu Thr
   65                  70                  75                  80
   Phe Thr Cys Asn Val Ala His Pro Ala Ser Lys Thr Lys Val Asp Lys
                   85                  90                  95
   Pro Val Pro Lys Arg Glu Asn Gly Arg Val Pro Arg Pro Pro Asp Cys
               100                 105                 110
   Pro Lys Cys Pro Ala Pro Glu Met Leu Gly Gly Pro Ser Val Phe Ile
           115                 120                 125
   Phe Pro Pro Lys Pro Lys Asp Thr Leu Leu Ile Ala Arg Thr Pro Glu
       130                 135                 140
   Val Thr Cys Val Val Val Asp Leu Asp Pro Glu Asp Pro Glu Val Gln
   145                 150                 155                 160
   Ile Ser Trp Phe Val Asp Gly Lys Gln Met Gln Thr Ala Lys Thr Gln
                   165                 170                 175
   Pro Arg Glu Glu Gln Phe Ala Gly Thr Tyr Arg Val Val Ser Val Leu
               180                 185                 190
   Pro Ile Gly His Gln Asp Trp Leu Lys Gly Lys Gln Phe Thr Cys Lys
           195                 200                 205
   Val Asn Asn Lys Ala Leu Pro Ser Pro Ile Glu Arg Thr Ile Ser Lys
       210                 215                 220
   Ala Arg Gly Gln Ala His Gln Pro Ser Val Tyr Val Leu Pro Pro Ser
   225                 230                 235                 240
   Arg Glu Glu Leu Ser Lys Asn Thr Val Ser Leu Thr Cys Leu Ile Lys
   245 250 255
   Asp Phe Tyr Pro Pro Asp Ile Asp Val Glu Trp Gln Ser Asn Gly Gln
   260 265 270
   Gln Glu Pro Glu Ser Lys Tyr Arg Thr Thr Pro Pro Gln Leu Asp Glu
   275 280 285
   Asp Gly Ser Tyr Phe Leu Tyr Ser Lys Leu Ser Val Asp Lys Ser Arg
   290 295 300
   Trp Gln Arg Gly Asp Thr Phe Ile Cys Ala Val Met His Glu Ala Leu
   305 310 315 320
   His Asn His Tyr Thr Gln Glu Ser Leu Ser His Ser Pro Gly Lys
   325 330 335
<210> 14
   <211> 333
   <212> PRT
   <213> Artificial Sequence
<220>
   <223> Aglycosyl canine immunoglobulin heavy chain type C Fc domain (HCC*)
<400> 14
   Ala Ser Thr Thr Ala Pro Ser Val Phe Pro Leu Ala Pro Ser Cys Gly
   1 5 10 15
   Ser Gln Ser Gly Ser Thr Val Ala Leu Ala Cys Leu Val Ser Gly Tyr
   20 25 30
   Ile Pro Glu Pro Val Thr Val Ser Trp Asn Ser Val Ser Leu Thr Ser
   35 40 45
   Gly Val His Thr Phe Pro Ser Val Leu Gln Ser Ser Gly Leu Tyr Ser
   50 55 60
   Leu Ser Ser Met Val Thr Val Pro Ser Ser Arg Trp Pro Ser Glu Thr
   65 70 75 80
   Phe Thr Cys Asn Val Ala His Pro Ala Thr Asn Thr Lys Val Asp Lys
   85 90 95
   Pro Val Ala Lys Glu Cys Glu Cys Lys Cys Asn Cys Asn Asn Cys Pro
   100 105 110
   Cys Pro Gly Cys Gly Leu Leu Gly Gly Pro Ser Val Phe Ile Phe Pro
           115                 120                 125
   Pro Lys Pro Lys Asp Ile Leu Val Thr Ala Arg Thr Pro Thr Val Thr
       130                 135                 140
   Cys Val Val Val Asp Leu Asp Pro Glu Asn Pro Glu Val Gln Ile Ser
   145                 150                 155                 160
   Trp Phe Val Asp Ser Lys Gln Val Gln Thr Ala Asn Thr Gln Pro Arg
                   165                 170                 175
   Glu Glu Gln Ser Ala Gly Thr Tyr Arg Val Val Ser Val Leu Pro Ile
               180                 185                 190
   Gly His Gln Asp Trp Leu Ser Gly Lys Gln Phe Lys Cys Lys Val Asn
           195                 200                 205
   Asn Lys Ala Leu Pro Ser Pro Ile Glu Glu Ile Ile Ser Lys Thr Pro
       210                 215                 220
   Gly Gln Ala His Gln Pro Asn Val Tyr Val Leu Pro Pro Ser Arg Asp
   225                 230                 235                 240
   Glu Met Ser Lys Asn Thr Val Thr Leu Thr Cys Leu Val Lys Asp Phe
                   245                 250                 255
   Phe Pro Pro Glu Ile Asp Val Glu Trp Gln Ser Asn Gly Gln Gln Glu
               260                 265                 270
   Pro Glu Ser Lys Tyr Arg Met Thr Pro Pro Gln Leu Asp Glu Asp Gly
           275                 280                 285
   Ser Tyr Phe Leu Tyr Ser Lys Leu Ser Val Asp Lys Ser Arg Trp Gln
       290                 295                 300
   Arg Gly Asp Thr Phe Ile Cys Ala Val Met His Glu Ala Leu His Asn
   305                 310                 315                 320
   His Tyr Thr Gln Ile Ser Leu Ser His Ser Pro Gly Lys
                   325                 330
<210> 15
   <211> 331
   <212> PRT
   <213> Artificial Sequence
<220>
   <223> Canine immunoglobulin heavy chain type D Fc domain (HCD*)
<400> 15
   Ala Ser Thr Thr Ala Pro Ser Val Phe Pro Leu Ala Pro Ser Cys Gly
   1               5                   10                  15
   Ser Thr Ser Gly Ser Thr Val Ala Leu Ala Cys Leu Val Ser Gly Tyr
               20                  25                  30
   Phe Pro Glu Pro Val Thr Val Ser Trp Asn Ser Gly Ser Leu Thr Ser
           35                  40                  45
   Gly Val His Thr Phe Pro Ser Val Leu Gln Ser Ser Gly Leu Tyr Ser
       50                  55                  60
   Leu Ser Ser Thr Val Thr Val Pro Ser Ser Arg Trp Pro Ser Glu Thr
   65                  70                  75                  80
   Phe Thr Cys Asn Val Val His Pro Ala Ser Asn Thr Lys Val Asp Lys
                   85                  90                  95
   Pro Val Pro Lys Glu Ser Thr Cys Lys Cys Ile Ser Pro Cys Pro Val
               100                 105                 110
   Pro Glu Ser Leu Gly Gly Pro Ser Val Phe Ile Phe Pro Pro Lys Pro
           115                 120                 125
   Lys Asp Ile Leu Arg Ile Thr Arg Thr Pro Glu Ile Thr Cys Val Val
       130                 135                 140
   Leu Asp Leu Gly Arg Glu Asp Pro Glu Val Gln Ile Ser Trp Phe Val
   145                 150                 155                 160
   Asp Gly Lys Glu Val His Thr Ala Lys Thr Gln Pro Arg Glu Gln Gln
                   165                 170                 175
   Phe Ala Ser Thr Tyr Arg Val Val Ser Val Leu Pro Ile Glu His Gln
               180                 185                 190
   Asp Trp Leu Thr Gly Lys Glu Phe Lys Cys Arg Val Asn His Ile Gly
           195                 200                 205
   Leu Pro Ser Pro Ile Glu Arg Thr Ile Ser Lys Ala Arg Gly Gln Ala
       210                 215                 220
   His Gln Pro Ser Val Tyr Val Leu Pro Pro Ser Pro Lys Glu Leu Ser
   225                 230                 235                 240
   Ser Ser Asp Thr Val Thr Leu Thr Cys Leu Ile Lys Asp Phe Tyr Pro
                   245                 250                 255
   Pro Glu Ile Asp Val Glu Trp Gln Ser Asn Gly Gln Pro Glu Pro Glu
               260                 265                 270
   Ser Lys Tyr His Thr Thr Ala Pro Gln Leu Asp Glu Asp Gly Ser Tyr
           275                 280                 285
   Phe Leu Tyr Ser Lys Leu Ser Val Asp Lys Ser Arg Trp Gln Gln Gly
       290                 295                 300
   Asp Thr Phe Thr Cys Ala Val Met His Glu Ala Leu Gln Asn His Tyr
   305                 310                 315                 320
   Thr Asp Leu Ser Leu Ser His Ser Pro Gly Lys
                   325                 330